(19) Europäisches Patentamt
European Patent Office
Office européen des brevets

(11) **EP 3 671 184 B1**

(12) **EUROPÄISCHE PATENTSCHRIFT**

(45) Veröffentlichungstag und Bekanntmachung des Hinweises auf die Patenterteilung:
**31.08.2022 Patentblatt 2022/35**

(21) Anmeldenummer: **19217925.7**

(22) Anmeldetag: **19.12.2019**

(51) Internationale Patentklassifikation (IPC):
$G01N\ 21/3504^{(2014.01)}$ $\quad$ $G01N\ 33/98^{(2006.01)}$
$A61B\ 5/08^{(2006.01)}$ $\quad$ $G01N\ 33/497^{(2006.01)}$

(52) Gemeinsame Patentklassifikation (CPC):
**G01N 21/3504; A61B 5/082; G01N 33/98**

(54) **ALKOHOLDETEKTIONSVORRICHTUNG MIT REDUNDANTEN MESSKANÄLEN UND VERFAHREN ZUM DETEKTIEREN VON ALKOHOL**

ALCOHOL DETECTION DEVICE WITH REDUNDANT MEASUREMENT CHANNELS AND METHOD FOR DETECTING ALCOHOL

DISPOSITIF DE DÉTECTION D'ALCOOL DOTÉ DE CANAUX DE MESURE REDONDANTS ET PROCÉDÉ POUR DÉTECTER D'ALCOOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2018 DE 102018009981**

(43) Veröffentlichungstag der Anmeldung:
**24.06.2020 Patentblatt 2020/26**

(73) Patentinhaber: **Dräger Safety AG & Co. KGaA 23560 Lübeck (DE)**

(72) Erfinder:
• **Stock, Burkhard**
**19217 Carlow (DE)**
• **Baesler, Malte**
**22941 Bargteheide (DE)**

(74) Vertreter: **Guthöhrlein, Gerhard Drägerwerk AG & Co. KGaA Moislinger Allee 53 - 55 23558 Lübeck (DE)**

(56) Entgegenhaltungen:
DE-B3-102011 106 410 $\quad$ US-A1- 2002 036 266
US-A1- 2018 116 555

**EP 3 671 184 B1**

**Beschreibung**

Wenn ein Mensch Alkohol zu sich genommen hat, so enthält die von ihm ausgeatmete Atemluft einen Anteil an Alkohol (Ethanol). Eine Alkoholdetektionsvorrichtung erkennt automatisch, ob ein Mensch (die Testperson) Alkohol zu sich genommen hat und dieser noch nicht abgebaut ist und daher die Testperson noch unter dem Einfluss von Alkohol steht. Die Testperson gibt eine Atemprobe in die Alkoholdetektionsvorrichtung ein, und die Alkoholdetektionsvorrichtung untersucht automatisch die Atemprobe auf Alkohol. Typischerweise umfasst eine solche Alkoholdetektionsvorrichtung eine Messkammer, in welche eine Gasprobe mit von der Testperson ausgeatmeter Atemluft verbracht wird und eine Sensorik. Beispielsweise bläst die Testperson in ein Mundstück, welches in Fluidverbindung mit der Messkammer steht.

Die Alkoholdetektionsvorrichtung soll mindestens erkennen und ausgeben, ob die eingegebene Atemprobe einen Alkoholgehalt oberhalb einer vorgegebenen Schranke, beispielsweise oberhalb einer vorgegebenen Nachweisgrenze, enthält oder nicht. Ist Alkohol detektiert, so wird bevorzugt die Testperson genauer untersucht. Möglich ist auch, dass die Alkoholdetektionsvorrichtung eine Alkoholkonzentration in der Atemluft misst und ausgibt.

Bekannt geworden sind unterschiedliche Arten, wie eine Alkoholdetektionsvorrichtung den Gehalt von Alkohol in einer Gasprobe misst, während diese Gasprobe sich in der Messkammer befindet. Ein aus DE10 2011 106 410 B3 oder US 2018/0116555 A1 bekannt gewordenes Prinzip ist, dass ein Infrarot-Strahl (IR-Strahl) in die Messkammer emittiert wird, die Gasprobe in der Messkammer durchdringt und auf einen Photosensor auftrifft, der abhängig von der Intensität des auftreffenden IR-Strahls einen Messwert erzeugt. Alkohol in der Gasprobe bewirkt eine Dämpfung und reduziert daher in einem bestimmten Wellenlängen-Bereich die Lichtintensität, was zu einem - im Vergleich zu einer alkoholfreien Gasprobe - veränderten Messwert führt. Die erfindungsgemäße Detektionsvorrichtung nutzt ebenfalls dieses Prinzip.

Der Erfindung liegt die Aufgabe zugrunde, eine Vorrichtung und ein Verfahren zur Detektion von Alkohol in einer Gasprobe bereitzustellen, welche mit größerer Zuverlässigkeit als bekannte Vorrichtungen und Verfahren arbeiten.

Die Aufgabe wird durch eine Vorrichtung mit den Merkmalen des Anspruchs 1 und durch ein Verfahren mit den Merkmalen des Anspruchs 11 gelöst.

Vorteilhafte Ausgestaltungen sind in den abhängigen Ansprüchen angegeben. Vorteilhafte Ausgestaltungen der Vorrichtung können auch vorteilhafte Ausgestaltungen des Verfahrens sein und umgekehrt.

Die erfindungsgemäße Vorrichtung umfasst

- eine Messkammer,
- eine erste IR-Strahlungsquelle und eine zweite IR-Strahlungsquelle,
- einen ersten IR-Detektor und einen zweiten IR-Detektor sowie
- ein signalverarbeitende Auswerteeinheit.

Die Messkammer vermag eine Gasprobe aufzunehmen, insbesondere eine auf Alkohol zu untersuchende Atemprobe oder sonstige Gasprobe oder auch eine von Alkohol freie Referenz-Gasprobe oder eine Alkohol enthaltende Referenz-Gasprobe.

Jede IR-Strahlungsquelle vermag jeweils einen IR-Strahl in die Messkammer zu emittieren. Jede IR-Detektor vermag jeweils einen Messwert zu erzeugen, und zwar abhängig von einem auftreffenden IR-Strahl, bevorzugt abhängig von der Lichtintensität eines auftreffenden IR-Strahls.

Die erfindungsgemäße Vorrichtung ist dazu ausgestaltet, wie folgt betrieben zu werden, und das erfindungsgemäße Verfahren umfasst die folgenden Schritte:

- Eine auf Alkohol zu prüfende Gasprobe wird in die Messkammer verbracht.

- Während sich die zu prüfende Gasprobe in der Messkammer befindet, emittieren die erste IR-Strahlungsquelle und die zweite IR-Strahlungsquelle jeweils einen IR-Strahl in die Messkammer.

- Der IR-Strahl von der ersten IR-Strahlungsquelle durchdringt die Messkammer und damit auch die zu prüfende Gasprobe in der Messkammer und trifft auf den ersten IR-Detektor auf.

- Der IR-Strahl von der zweiten IR-Strahlungsquelle durchdringt die Messkammer und damit auch die zu prüfende Gasprobe in der Messkammer und trifft auf den zweiten IR-Detektor auf.

- Der erste IR-Detektor erzeugt abhängig von dem auftreffenden IR-Strahl einen ersten Messwert.

- Der zweite IR-Detektor erzeugt abhängig von dem auftreffenden IR-Strahl einen zweiten Messwert.

- Die Auswerteeinheit entscheidet automatisch, ob die Gasprobe in der Messkammer Alkohol enthält oder nicht. Für diese Entscheidung verwendet die Auswerteeinheit mindestens die beiden Messwerte von den beiden IR-Sensoren, die erzeugt worden sind, während sich die zu untersuchende Gasprobe in der Messkammer befindet.

Die Messkammer umgibt die zu untersuchende Gasprobe und isoliert die Gasprobe von der Umgebungsluft.

Eine Detektionsvorrichtung mit IR-Strahlungsquellen und IR-Detektoren benötigt - im Gegensatz etwa zu einer Detektionsvorrichtung mit Halbleitersensoren - keine leistungsstarke Heizvorrichtung, um einen Halbleiter auf Reaktionstemperatur zu bringen, und ist in vielen Fällen zuverlässiger als eine Detektionsvorrichtung mit einem elektrochemischen Sensor. Außerdem braucht eine erfindungsgemäße IR-Detektionsvorrichtung keine Chemikalie, die mit Alkohol reagieren kann und durch die Reaktion Alkohol anzeigt. Eine solche Chemikalie verwenden zu müssen hat die Nachteile, dass die Chemikalie in der Regel von Zeit zu Zeit ersetzt werden muss und / oder gefährlich sein kann.

Die Erfindung stellt zwei unabhängig voneinander arbeitende Detektionseinheiten bereit, nämlich eine erste Detektionseinheit mit der ersten IR-Strahlungsquelle und dem ersten IR-Detektor sowie eine zweite Detektionseinheit mit der zweiten IR-Strahlungsquelle und dem zweiten IR-Detektor. Jede Detektionseinheit liefert jeweils mindestens einen Messwert. Die Auswerteeinheit steht mit diesen beiden Detektionseinheiten in einer Datenverbindung und empfängt beide Messwerte. Weil erfindungsgemäß zwei unabhängig voneinander arbeitende Detektionseinheiten verwendet werden, steigt die Zuverlässigkeit eines von der Detektionsvorrichtung gelieferten Ergebnisses verglichen mit einer Detektionsvorrichtung, die nur eine einzige Detektionseinheit aufweist.

Ermöglicht wird, dass die Auswerteeinheit die beiden oder wenigstens zwei Messwerte von den beiden Detektionseinheiten - oder zwei Signale, die von den Messwerten abhängen und jeweils ein Maß für die Alkoholkonzentration in der Gasprobe sind - miteinander vergleicht und abhängig von dem Vergleich nicht nur die Entscheidung fällt, ob die Gasprobe Alkohol enthält oder nicht, sondern zusätzlich entscheidet, ob dieses Ergebnis ausreichend zuverlässig ist oder nicht. Ermöglicht wird, dass die beiden Detektionseinheiten zwar die gleiche Empfindlichkeit für Ethanol aufweisen, aber unterschiedliche Empfindlichkeiten für mindestens eine andere Substanz, welche in einer Gasprobe auftreten kann. Dann vermag die Detektionsvorrichtung Alkohol von dieser anderen Substanz in der Gasprobe zu unterscheiden.

Weiterhin wird dank der beiden Detektionseinheiten Redundanz bereitgestellt: Falls eine IR-Strahlungsquelle oder ein IR-Detektor ausfallen, so kann die Detektionsvorrichtung immer noch eine Gasprobe in der Messkammer prüfen.

In vielen Fällen liefert die Detektionsvorrichtung dank der beiden redundanten Detektionseinheiten ein beweiskräftiges und / oder gerichtsfestes Ergebnis.

Erfindungsgemäß nutzen diese beiden Detektionseinheiten dieselbe Messkammer. Dadurch wird die Notwendigkeit erspart, eine zu untersuchende Gasprobe auf zwei Messkammern aufzuteilen oder gar zwei Gasproben bereitzustellen. Weil dieselbe Gasprobe in derselben Messkammer untersucht wird, lassen sich die Messwerte oder Signale der beiden Detektionseinheiten mit höherer Zuverlässigkeit miteinander vergleichen, als wenn zwei Detektionseinheiten mit zwei unterschiedlichen Messkammern verwendet werden würde. Außerdem ermöglicht es das erfindungsgemäße Merkmal, dass dieselbe Messkammer für beide Detektionseinheiten verwendet wird, die Detektionsvorrichtung kompakter und mit geringerem Platzbedarf auszugestalten, verglichen mit einer Detektionsvorrichtung umfassend zwei getrennte Messkammern. Insbesondere dieses Merkmal erleichtert es, eine von einem Menschen in einer Hand tragbare und trotzdem zuverlässige Detektionsvorrichtung bereitzustellen.

Erfindungsgemäß vermag die Detektionsvorrichtung automatisch zu entscheiden, ob eine zu untersuchende Gasprobe in der Messkammer Alkohol enthält oder nicht. In einer Ausgestaltung vermag die Detektionsvorrichtung zusätzlich den Gehalt oder die Konzentration von Alkohol (Ethanol) in dieser Gasprobe quantitativ zu bestimmen.

Bevorzugt arbeiten die beiden Detektionseinheiten und insbesondere die beiden IR-Detektoren unabhängig voneinander. Die Ergebnisse des einen IR-Detektors beeinflussen nicht die Ergebnisse des anderen IR-Detektors. Bevorzugt bündelt die Detektionsvorrichtung einen IR-Strahl von einer IR-Strahlungsquelle dergestalt, dass dieser IR-Strahl ausschließlich oder zumindest im Wesentlichen nur auf den dieser IR-Strahlungsquelle zugeordneten IR-Detektor auftrifft und nicht auf den oder einen anderen IR-Detektor. Der von der ersten IR-Strahlungsquelle emittierte IR-Strahl trifft bevorzugt ausschließlich oder fast ausschließlich auf den ersten IR-Detektor, der von der zweiten IR-Strahlungsquelle emittierte IR-Strahl ausschließlich oder fast ausschließlich auf den zweiten IR-Detektor. Die beiden Strahlengänge der beiden IR-Strahlen beeinflussen sich idealerweise nicht.

Bevorzugt ist die Messkammer spiegelsymmetrisch bezüglich einer Mittelebene aufgebaut. Bevorzugt befindet sich die erste Detektionseinheit mit der ersten IR-Strahlungsquelle und dem ersten IR-Detektor auf der einen Seite dieser Mittelebene, und die zweite Detektionseinheit mit der zweiten IR-Strahlungsquelle und den zweiten IR-Detektor befindet sich auf der anderen Seite. Diese Ausgestaltung ermöglicht eine besonders einfache Konstruktion.

Möglich ist, dass die beiden IR-Detektoren gleichartig aufgebaut sind und eine gleichartige spektrale Empfindlichkeit aufweisen. In einer bevorzugten Ausgestaltung umfasst hingegen jeder IR-Detektor jeweils einen Wellenlängen-Filter und den eigentlichen Photosensor. Jeder Wellenlängen-Filter vermag einen auftreffenden IR-Strahl so zu filtern, dass ein IR-Teilstrahl in einem bestimmten Wellenlängen-Bereich diesen Wellenlängen-Filter passiert und auf den Photosensor auftrifft. Anteile des auf den Filter auftreffenden IR-Strahls außerhalb dieses Wellenlängen-Bereichs werden herausgefiltert oder wenigstens abgeschwächt. Der Photosensor erzeugt abhängig von einem auftreffenden IR-Teilstrahl einen Messwert - bevorzugt: abhängig von der Lichtintensität des auftreffenden IR-Teilstrahls.

Bevorzugt lässt der erste Wellenlängen-Filter, der zum ersten IR-Detektor gehört, einen IR-Teilstrahl in einem ersten Wellenlängen-Bereich passieren. Der zweite Wellenlängen-Filter, der zum zweiten IR-Detektor gehört, lässt einen IR-Teilstrahl in einem zweiten Wellenlängen-Bereich passieren. Diese beiden Wellenlängen-Bereiche unterscheiden sich bevorzugt voneinander. Bevorzugt umfasst jeder Wellenlängen-Bereich eines Wellenlängen-Filters einen Teilbereich, der von Ethanol in der Luft hinreichend stark absorbiert wird. Die beiden Wellenlängen-Bereiche lassen sich so einstellen, dass die beiden Detektionseinheiten zwar die gleiche Empfindlichkeit für Ethanol aufweisen, also bei Alkohol in der Gasprobe etwa die gleichen Messwerte liefern, solange die Alkoholkonzentration in einem vorgegebenen Bereich liegt, jedoch unterschiedliche Empfindlichkeiten für mindestens eine andere Substanz, die ebenfalls in der Gasprobe und damit in der Messkammer enthalten sein kann. Diese Ausgestaltung reduziert weiter die Empfindlichkeit der Detektionsvorrichtung gegenüber Substanzen, welche ein Ergebnis der Detektionsvorrichtung beeinflussen könnten, insbesondere Alkohol vortäuschen oder aber verdecken könnten.

Diese Ausgestaltung mit den Wellenlängen-Filtern ermöglicht es auf besonders einfache Weise, zwei gleichartige Photosensoren für die beiden IR-Detektoren bereitzustellen. Dies reduziert die Varianz. Die unterschiedlichen Empfindlichkeiten lassen sich ausschließlich durch unterschiedliche oder ansteuerbare Wellenlängen-Filter und / oder indem die Auswerteeinheit die Messwerte der IR-Detektoren geeignet auswertet erzielen.

In einer Ausgestaltung lässt sich mindestens ein Wellenlängen-Filter, bevorzugt jeder Wellenlängen-Filter, wahlweise in einem ersten Modus oder in einem zweiten Modus betreiben. Im ersten Modus lässt der Wellenlängen-Filter einen IR-Teilstrahl im ersten Wellenlängen-Bereich passieren, im zweiten Modus einen IR-Teilstrahl im zweiten Wellenlängen-Bereich. Diese Ausgestaltung ermöglicht es, wahlweise den ersten Wellenlängen-Filter im ersten Modus und den zweiten Wellenlängen-Filter im zweiten Modus oder den ersten Wellenlängen-Filter im zweiten Modus und den zweiten Wellenlängen-Filter im ersten Modus zu betreiben.

In einer Realisierung dieser Ausgestaltung besitzt der oder jeder Wellenlängen-Filter, der in zwei Modi betreibbar ist, ein erstes Segment, welches einen IR-Teilstrahl im ersten Wellenlängen-Bereich passieren lässt, und ein zweites Segment, welches einen IR-Teilstrahl im zweiten Wellenlängen-Bereich passieren lässt. Der Wellenlängen-Filter lässt sich relativ zu dem zugeordneten Photosensor bewegen, sodass ein auftreffender IR-Strahl je nach Position des Wellenlängen-Filters durch das erste Segment oder durch das zweite Segment gefiltert wird. Der Wellenlängen-Filter ist z. B. drehbar oder linear verschiebbar angeordnet. In einer anderen Realisierung dieser Ausgestaltung lässt sich der Wellenlängen-Filter umschalten, indem eine anliegende Steuerspannung entsprechend verstellt wird

Ermöglicht wird, dass bei der Untersuchung einer Gasprobe in der Messkammer jeder IR-Detektor nacheinander jeweils zwei Messwerte erzeugt, nämlich einen ersten Messwert, während der Wellenlängen-Filter dieses IR-Detektors im ersten Modus ist, und einen zweiten Messwert, während der Wellenlängen-Filter dieses IR-Detektors im zweiten Modus ist. Die Auswerteeinheit fällt die Entscheidung, ob die Gasprobe Alkohol enthält oder nicht, abhängig mindestens von den beiden ersten Messwerte und von den beiden zweiten Messwerten. Diese Ausgestaltung steigert weiter die Zuverlässigkeit der Detektionsvorrichtung. Weiterhin ermöglicht es diese Ausgestaltung auf besonders einfache Weise, zwei gleichartige IR-Detektoren bereitzustellen.

Möglich ist, dass die Detektionsvorrichtung automatisch den oder jeden Wellenlängen-Filter von dem einen Modus in den anderen Modus umschaltet, während sich eine zu untersuchende Gasprobe in der Messkammer befindet. Möglich ist auch, dass eine entsprechende Benutzereingabe den Schritt auslöst, den oder jeden Wellenlängen-Filter umzuschalten.

Erfindungsgemäß durchdringt ein IR-Strahl von der ersten IR-Strahlungsquelle die Messkammer und erreicht den ersten IR-Detektor. Ein IR-Strahl von der zweiten IR-Strahlungsquelle durchdringt ebenfalls die Messkammer und erreicht den zweiten IR-Detektor. Möglich ist, dass diese IR-Strahlen auf direktem Wege den jeweiligen IR-Detektor erreichen. Bevorzugt wird hingegen jeder IR-Strahl auf seinem Weg in der Messkammer von der jeweiligen IR-Strahlungsquelle zu dem jeweiligen IR-Detektor mindestens einmal gespiegelt, bevorzugt mehrmals gespiegelt. Diese Ausgestaltung vergrößert die Länge des Strahlengangs, den ein IR-Strahl auf dem Weg von der IR-Strahlungsquelle zu dem IR-Detektor zurücklegt, und zwar bevorzugt um ein Mehrfaches. Auf diese Weise wird bei gleicher Abmessung der Messkammer eine längere Strecke erzielt, auf welcher die zu untersuchende Gasprobe Einfluss auf die Lichtintensität nehmen kann (größere Absorptionslänge). Weiterhin durchdringt jeder IR-Strahl einen größeren Teil des Volumens der Messkammer, sodass ein Ergebnis der Detektionsvorrichtung auch dann eine höhere Zuverlässigkeit aufweist, wenn Alkohol ungleichmäßig in der Messkammer verteilt sein kann, verglichen mit einer Ausgestaltung der Detektionsvorrichtung ohne Spiegel. Erfindungsgemäß umfasst die Detektionsvorrichtung zwei Detektionseinheiten, umfassend jeweils eine IR-Strahlungsquelle und jeweils einen IR-Detektor. Die beiden IR-Strahlungsquellen senden jeweils einen IR-Strahl in dieselbe Messkammer aus, und auf die beiden IR-Detektoren trifft jeweils ein IR-Strahl aus derselben Messkammer auf. Durch die Spiegel ergibt sich der weitere Vorteil, dass derselbe Spiegel oder dieselbe Anordnung mit mehreren Spiegeln für beide Detektionseinheiten verwendet wird. Nicht erforderlich ist es, für jede Detektionseinheit jeweils eine eigene Spiegel-Anordnung bereitzustellen. Dies reduziert die Anzahl der erforderlichen Bestandteile und damit die Abmessung und / oder das Gewicht der Detektionsvorrichtung, ohne deren Zuverlässigkeit zu reduzieren.

Erfindungsgemäß durchdringen beide IR-Strahlen dieselbe Messkammer. Dies hat insbesondere folgenden Vorteil gegenüber einer denkbaren Ausgestaltung mit zwei Messkammern, die von jeweils einen IR-Strahl durchdrungen werden: Entweder wird die Detektionsvorrichtung mit zwei Messkammern deutlich größer als die erfindungsgemäße Detektionsvorrichtung mit einer von beiden Detektionseinheiten genutzten Messkammer. Oder jede der beiden Messkammern ist kleiner als die Messkammer der erfindungsgemäßen Detektionsvorrichtung. Dann kann jeder IR-Strahl nur eine geringere Absorptionslänge erzielen als die IR-Strahlen der erfindungsgemäßen Detektionsvorrichtung. Außerdem erzeugt eine erfindungsgemäße Detektionsvorrichtung in vielen Fällen bei gleichem Energieverbrauch eine höhere Lichtintensität der emittierte IR-Strahlen als andere Detektionsvorrichtung.

Erfindungsgemäß ist an zwei gegenüberliegenden Wänden der Messkammer jeweils mindestens ein Spiegel angeordnet, sodass die Messkammer mindestens zwei Spiegel aufweist. Ein IR-Strahl wird auf seinem Weg von einer IR-Strahlungsquelle zu einem IR-Detektor daher bevorzugt mindestens zweimal gespiegelt, besonders bevorzugt zwischen viermal und achtmal, insbesondere sechsmal. Erfindungsgemäß liegen die beiden Wände mit den beiden Spiegeln und damit die beiden Spiegel einander gegenüber, sodass sich eine Gasprobe in der Messkammer zwischen diesen beiden Spiegeln befindet. Bevorzugt liegt die Absorptionslänge zwischen 350 mm und 450 mm, besonders bevorzugt bei etwa 400 mm. Der Abstand zwischen den beiden Spiegeln liegt bevorzugt zwischen 50 mm und 60 mm. Wie oft ein IR-Strahl reflektiert wird, hängt vom Abstand zwischen den Spiegeln ab. Bevorzugt ist mindestens ein Spiegel, besonders bevorzugt beide Spiegel, als jeweils ein Konkavspiegel ausgestaltet. Diese Ausgestaltung lenkt einen auftreffenden IR-Strahl stärker zur Mitte der Messkammer hin - verglichen mit einem planen Spiegel. Dies erleichtert es, zuverlässig eine gewünschte Absorptionslänge zu erzielen. Wie oft ein IR-Strahl reflektiert wird, hängt zusätzlich von den Brennweiten der beiden Konkavspiegel ab.

Erfindungsgemäß ist jedem IR-Detektor jeweils eine IR-Strahlungsquelle zugeordnet. Ein IR-Strahl von dieser zugeordneten IR-Strahlungsquelle erreicht den IR-Detektor. Bevorzugt wird der IR-Strahl auf dem Weg von der IR-Strahlungsquelle zu dem IR-Detektor mindestens einmal an einem Spiegel reflektiert, besonders bevorzugt mindestens einmal an einem ersten Spiegel und mindestens einmal an einem zweiten Spiegel, wobei die beiden Spiegel erfindungsgemäß einander gegenüberliegen. In einer Ausgestaltung sind der IR-Detektor und die IR-Strahlungsquelle an zwei einander gegenüberliegenden Wänden mit jeweils einem Spiegel angeordnet, sodass die Messkammer und damit die Gasprobe in der Messkammer sich zwischen den Spiegeln befindet.

Ein IR-Strahl wird auf seinem Weg von der IR-Strahlungsquelle zu dem IR-Detektor also mindestens zweimal, bevorzugt viermal oder sogar sechsmal, gespiegelt. Die Absorptionslänge beträgt dann das Dreifache, Fünffache oder sogar Siebenfache des Abstands zwischen den beiden gegenüberliegenden Spiegeln.

In einer Ausgestaltung sind die beiden IR-Strahlungsquellen an zwei gegenüberliegenden Wänden der Messkammer angeordnet. Diese Ausgestaltung reduziert in manchen Fällen die Gefahr, dass die beiden IR-Strahlungsquellen sich in unerwünschter Weise gegenseitig beeinflussen. In einer anderen Ausgestaltung sind die beiden IR-Strahlungsquellen an derselben Wand der Messkammer angeordnet, bevorzugt mit einem Abstand voneinander. Diese Ausgestaltung verkürzt in vielen Fällen die erforderliche Länge von Leitungen zwischen der Auswerteeinheit und den beiden IR-Strahlungsquellen.

Erfindungsgemäß sind die beiden IR-Detektoren an zwei gegenüberliegenden Wänden der Messkammer angeordnet. In einer anderen, nicht beanspruchten, Ausgestaltung sind die beiden IR-Detektoren an derselben Wand der Messkammer angeordnet, bevorzugt mit einem Abstand zueinander. In einer Fortbildung dieser Ausgestaltung sind die beiden IR-Detektoren an derselben ersten Wand der Messkammer angeordnet und die beiden IR-Strahlungsquellen an derselben zweiten Wand der Messkammer, wobei die erste Wand und die zweite Wand einander gegenüberliegen, sodass eine Gasprobe in der Messkammer sich zwischen diesen beiden Wänden befindet.

In einer Fortbildung dieser Ausgestaltung sind die beiden IR-Strahlungsquellen und / oder die beiden IR-Detektoren in Spiegeln an den Wänden der Messkammer integriert. Beispielsweise sind die beiden IR-Strahlungsquellen in einen Spiegel integriert, und die beiden IR-Detektoren sind in einen gegenüberliegenden Spiegel integriert. Dies ist aber nicht beansprucht.

In einer Fortbildung der Ausgestaltung mit den Spiegeln ist die Messkammer als eine Herriott-Zelle mit zwei einander gegenüberliegenden Konkavspiegeln ausgebildet und ist spiegelsymmetrisch bezogen auf eine Mittelebene. Bevorzugt liegen die beiden Brennpunkte der beiden einander gegenüberliegenden Konkavspiegel auf dieser Mittelebene. Diese Mittelebene stellt somit eine übereinstimmende optische Achse der beiden Konkavspiegel bereit. Vorzugsweise ist die Messkammer gasdicht ausgebildet, sodass die Untersuchung der Gasprobe nicht durch Gas, das zusätzlich in die Messkammer gelangt, oder durch aus der Messkammer entweichendes Gas der Gasprobe verfälscht wird. Vorzugsweise kann die zu untersuchende Gasprobe ausschließlich durch einen Einlass in die Messkammer gelangen. Vorzugsweise kann die zu untersuchende Gasprobe ausschließlich durch einen Auslass wieder die Messkammer verlassen. Vorzugsweise ist dieser Auslass durch ein Rückschlagventil oder ein sonstiges entsprechend geeignetes Element gesichert, so dass zwar Gas durch den Auslass hindurch die Messkammer verlassen kann, aber kein Gas durch den Auslass in die Messkammer gelangen kann, insbesondere keine Umgebungsluft. Möglich ist, dass dieselbe Öffnung in einer Wand der Messkammer sowohl zum Einlass als auch zum Auslass gehört.

Bevorzugt liegt die maximale Abmessung der Messkammer zwischen 80 mm und 120 mm, besonders bei etwa 100 mm.

Erfindungsgemäß vermag die Messkammer eine auf Alkohol zu untersuchende Gasprobe aufzunehmen. Diese Gasprobe wird beispielsweise von einer Testperson abgegeben, insbesondere durch Ausatmen, und die Testperson verbringt selber die Gasprobe in die Messkammer. Möglich ist auch, dass die Detektionsvorrichtung eine Fördereinheit aufweist, welche die zu untersuchende Gasprobe und / oder eine Referenz-Gasprobe in die Messkammer fördert.

In einer Ausgestaltung vermag die Detektionsvorrichtung die Messkammer auszuspülen, insbesondere eine bereits untersuchte Gasprobe aus der Messkammer zu entfernen und durch eine Referenz-Gasprobe zu ersetzen, welche keinen Alkohol enthält oder aber welche Alkohol in einer bekannten Konzentration enthält. Möglich ist, dass die Detektionsvorrichtung automatisch die untersuchte Gasprobe oder das seit Längerem in der Messkammer befindliche Gas durch eine neue zu untersuchende Gasprobe oder aber durch eine Referenz-Gasprobe ersetzt, beispielsweise nach jeder Untersuchung einer Gasprobe oder in vorgegebenen Zeitabständen. Möglich ist auch, dass die Detektionsvorrichtung als Reaktion auf eine entsprechende Benutzereingabe die untersuchte Gasprobe durch eine Referenz-Gasprobe ersetzt.

Möglich ist, dass diese Referenz-Gasprobe von einer externen Förderereinheit oder beispielsweise von einer Pressluftflasche zugeführt wird.

In einer bevorzugten Realisierung dieser Ausgestaltung umfasst die Detektionsvorrichtung hingegen eine eigene Förderereinheit, insbesondere eine Pumpe, welche eine Referenz-Gasprobe in die Messkammer oder aus der Messkammer zu fördern vermag, sowie einen Auslass, durch welchen die untersuchende Gasprobe aus der Messkammer entweichen kann. Bevorzugt steuert die Auswerteeinheit diese Förderereinheit an, und die Förderereinheit bleibt so lange eingeschaltet und fördert so lange Gas, bis die Messkammer ausschließlich mit der Referenz-Gasprobe gefüllt ist. In einer Realisierung schaltet die Auswerteeinheit die Förderereinheit für eine vorgegebene Zeitspanne ein und schaltet sie danach wieder aus. In einer anderen Ausgestaltung misst ein Durchflusssensor am Einlass den Volumenstrom, und die Auswerteeinheit berechnet aufgrund von Messwerten des Durchflusssensors, welche Menge von Gas die Förderereinheit bislang in die Messkammer geleitet hat (Integration über den Volumenstrom), und schaltet die Förderereinheit wieder ab, wenn diese bislang geförderte Menge das Volumen der Messkammer erreicht oder überstiegen hat, beispielsweise nachdem mindestens ein 1 l Gas in die Messkammer gefördert wurde. Der Durchflusssensor kann auch den Fluss von Gas aus der Messkammer heraus messen.

Die Referenz-Gasprobe kann eine Gasprobe sein, die frei von Alkohol ist. Möglich ist, dass eine solche alkoholfreie Gasprobe aus der Umgebungsluft entnommen wird. In dem Einlass in die Messkammer kann ein Filter vorgesehen sein, welcher Partikel aus der einströmenden Luft oder der eingegebenen Atemprobe herausfiltert. Eine Referenz-Gasprobe, die eine bekannte Konzentration von Alkohol enthält, wird beispielsweise bei einer optionalen Kalibrierung oder Justierung der Detektionsvorrichtung zugeführt, welche beispielhaft weiter unten beschrieben wird.

Die Ausgestaltung, dass die Detektionsvorrichtung eine untersuchte Gasprobe durch eine alkoholfreie Referenz-Gasprobe in der Messkammer ersetzen kann, ermöglicht folgende Betriebsart der erfindungsgemäßen Detektionsvorrichtung:

- Wenn die Messkammer mit der alkoholfreien Gasprobe gefüllt ist, so emittiert jede IR-Strahlungsquelle jeweils einen IR-Strahl in die Messkammer. Jeder IR-Detektor erzeugt jeweils einen Messwert. Die Lichtintensität dieses IR-Strahls wird nicht durch Alkohol in der Gasprobe reduziert. Daher wird der Messwert vom ersten IR-Detektor als erster Null-Messwert verwendet, der Messwert vom zweiten IR-Detektor als zweiter Null-Messwert.
- Anschließend wird die Messkammer mit der zu untersuchenden Gasprobe gefüllt. Wiederum emittiert jede IR-Strahlungsquelle jeweils einen IR-Strahl in die Messkammer. Jeder IR-Detektor erzeugt jeweils einen Messwert. Mindestens dann, wenn die zu untersuchende Gasprobe Alkohol enthält, ist in der Regel ein Messwert kleiner als der entsprechende Null-Messwert von demselben IR-Detektor, weil Alkohol IR-Licht absorbiert und damit abschwächt.
- Möglich ist auch, dass zunächst die zu untersuchende Gasprobe und dann die alkoholfreie Gasprobe in die Messkammer geleitet wird und daher zunächst die Messwerte und dann die Null-Messwerte erzeugt werden.
- Die Auswerteeinheit erzeugt abhängig vom ersten Null-Messwert und vom ersten Messwert (Messwert, den der erste IR-Detektor erzeugt hat, während die Messkammer mit der zu untersuchenden Gasprobe gefüllt ist) ein erstes Signal. Die Auswerteeinheit erzeugt abhängig vom zweiten Null-Messwert und vom zweiten Messwert ein zweites Signal. Jedes Signal ist ein Maß für die Konzentration von Ethanol in der Gasprobe. Die Auswerteeinheit vergleicht diese beiden Signale miteinander und erzeugt abhängig vom Ergebnis des Vergleichs ein Untersuchungsergebnis.

Weil die erfindungsgemäße Detektionsvorrichtung zwar zwei bevorzugt unabhängig voneinander arbeitende Detektionseinheiten umfasst, diese beiden Detektionseinheiten aber dieselbe Messkammer nutzen, braucht auch nur eine Messkammer geleert zu werden. Dies ist ein weiterer Vorteil im Vergleich zu einer Detektionsvorrichtung mit zwei getrennten Messkammern.

Jede IR-Strahl durchläuft auf seinem Weg von einer IR-Strahlungsquelle zu dem zugeordneten IR-Detektor einen Strahlengang und wird auf diesem Strahlengang mehr oder weniger stark von Gas in der Messkammer absorbiert. Daher erzielt jeder IR-Strahl jeweils eine Absorptionslänge. Bevorzugt ist die Detektionsvorrichtung so ausgestaltet, dass die beiden IR-Strahlen idealerweise dieselbe Absorptionslänge erzielen. In der Praxis können die tatsächlich erzielten Absorptionslängen aber beispielsweise durch Fertigungs- und MontageUngenauigkeiten voneinander differieren. Das gerade beschriebene Vorgehen, vor oder nach jeder Untersuchung einer Gasprobe die Messkammer zu leeren, reduziert den Einfluss von unterschiedlichen Absorptionslänge auf die Messungen.

Diese Ausgestaltung lässt sich kombinieren mit der Ausgestaltung, dass die beiden IR-Detektoren zwar die gleiche Empfindlichkeit für Ethanol in der Gasprobe aufweisen, aber unterschiedliche Empfindlichkeiten für mindestens eine weitere mögliche Substanz. Falls die beiden Signale innerhalb einer Toleranz übereinstimmen, so ist das Messergebnis zuverlässig, und zwar sowohl dann, wenn Alkohol detektiert wird, als auch dann, wenn die Nichtexistenz von Alkohol in der Gasprobe detektiert wird. Falls die beiden Signale stärker als die Toleranz voneinander abweichen, so liefert die Auswerteeinheit bevorzugt abhängig von den beiden Signale mindestens eines der folgenden Ergebnisse:

- Die Gasprobe enthält mindestens eine weitere Substanz, welche die Lichtintensität des einen IR-Strahls stärker abschwächt als die Lichtintensität des anderen IR-Strahl.
- Die Messkammer ist einer störenden elektromagnetischen Strahlung von außen ausgesetzt, welche die IR-Strahlen der beiden IR-Strahlungsquellen überlagert.
- Eine IR-Strahlungsquelle und / oder ein IR-Detektor und / oder eine Datenverbindung sind ausgefallen.

Die Detektionsvorrichtung vermag in einer dieser Situationen oft nicht mit ausreichender Sicherheit Alkohol von anderen Substanzen in der Messkammer zu unterscheiden. In manchen Fällen ist es möglich, die Messkammer auszuspülen, indem eine alkoholfreie Gasprobe in die Messkammer geleitet wird, dann erneut eine Gasprobe in die Messkammer geleitet und diese untersucht wird, wobei dann keine störenden Substanzen mehr in der Messkammer vorhanden sind.

Die Ausgestaltung, dass regelmäßig, beispielsweise nach oder auch vor jeder Untersuchung einer Gasprobe, eine alkoholfreie Referenz-Gasprobe in die Messkammer geleitet wird und zwei Null-Messwerte erzeugt werden, hat insbesondere folgenden Vorteil: Falls eine Eigenschaft einer IR-Spannungsquelle oder eines IR-Detektors sich allmählich verändert (z.B. aufgrund von Parameter-Drift oder nachlassender Spannungsversorgung oder Verschmutzung), so lässt sich diese allmähliche Veränderung rechnerisch und mit geringem Aufwand kompensieren. Ausreichend ist, dass die Eigenschaften in derjenigen Zeitspanne ausreichend konstant bleiben, während der sich die Referenz-Gasprobe und danach oder davor die zu untersuchende Gasprobe in der Messkammer befinden. Dies ist in der Regel der Fall, weil diese Zeitspanne im Bereich unter einer Minute oder im Bereich von wenigen Minuten liegt.

In einer Ausgestaltung hängen die beiden Signale, welche die Auswerteeinheit abhängig von den beiden Messwerten und von den beiden Null-Messwerten berechnet, von der absoluten oder prozentualen Abschwächung ab, welche Alkohol oder eine sonstige Substanz in der zu untersuchenden Gasprobe auf die Lichtintensität des jeweiligen IR-Strahls bewirkt.

In einer Fortbildung dieser Ausgestaltung hängen die beiden Signale zusätzlich von zwei Kalibrierungsfaktoren ab. Bevorzugt werden diese Kalibrierungsfaktoren vorgegeben oder aber vorab erzeugt. Bei dieser Vorab-Erzeugung erzeugen die beiden IR-Detektoren einerseits so wie gerade beschrieben zwei Null-Messwerte, während sich eine alkoholfreie Referenz-Gasprobe in der Messkammer befindet. Außerdem wird eine weitere Referenz-Gasprobe in die Messkammer geleitet, wobei diese weitere Referenz-Gasprobe Alkohol enthält, bevorzugt in einer Konzentration, die typischerweise in der ausgeatmeten Atemluft eines Menschen auftritt, der Alkohol konsumiert hat. Die beiden IR-Detektoren erzeugen zwei Referenz-Messwerte, während die Messkammer mit dieser alkoholhaltige Referenz-Gasprobe gefüllt ist.

Um später eine Gasprobe auf Alkohol zu untersuchen, erzeugt die Auswerteeinheit so wie oben beschrieben zwei Signale, die von den beiden Messwerten und von den beiden Null-Messwerten sowie von den beiden Kalibrierungsfaktoren abhängen. Hierbei wendet die Auswerteeinheit eine abgespeicherte Berechnungsvorschrift an, welche die beiden Kalibrierungsfaktoren enthält. Bei der Kalibrierung werden die beiden Kalibrierungsfaktoren so eingestellt, dass die Rechenvorschrift angewendet auf den ersten Referenz-Messwert, den ersten Null-Messwert und den ersten Kalibrierungsfaktor dasselbe Signal liefert wie angewendet auf den zweiten Referenz-Messwert, den zweiten Null-Messwert und den zweiten Kalibrierungsfaktor. Dadurch haben die beiden IR-Detektoren die gleiche Empfindlichkeit für Ethanol. Bevorzugt werden so wie oben beschrieben zwei unterschiedliche Wellenlängen-Filter verwendet.

Diese Ausgestaltung mit dem Kalibrierungsfaktoren und bevorzugt den unterschiedlichen Wellenlängen-Filtern reduziert weiter den Einfluss von möglicherweise unterschiedlichen Absorptionslängen der beiden IR-Strahlen. In manchen Fällen wird ermöglicht, bei der Herstellung der Detektionsvorrichtung eine größere Fertigungs- und Montagetoleranz vorzugeben - verglichen mit einer Ausgestaltung, bei der beide Absorptionslänge exakt übereinstimmen. Erfindungsgemäß umfasst die Detektionsvorrichtung zwei Detektionseinheiten mit jeweils einer IR-Strahlungsquelle und einem IR-Detektor. Möglich ist, dass die Detektionsvorrichtung drei oder sogar noch mehr Detektionseinheiten mit jeweils einer IR-Strahlungsquelle und einem IR-Detektor umfasst, welche alle dieselbe Messkammer und dieselbe Spiegel-Anordnung nutzen, wobei die IR-Strahlungsquellen und die IR-Detektoren jeweils gleichartig aufgebaut sein können.

Die erfindungsgemäße Detektionsvorrichtung lässt sich für eine Untersuchung verwenden, ob eine Atemprobe von einer Testperson Alkohol enthält oder nicht. Die Detektionsvorrichtung lässt sich auch für andere Anwendungen verwenden, bei der eine Gasprobe darauf untersucht werden soll, ob sie Alkohol enthält, z.B. die Prüfung, ob ein Behälter, der eine alkoholhaltige Flüssigkeit enthält, dicht ist oder ob Alkoholdampf austritt.

Im Folgenden wird die Erfindung anhand eines Ausführungsbeispiels beschrieben. Hierbei zeigen

Figur 1    eine Ausgestaltung einer nicht beanspruchten Detektionsvorrichtung;
Figur 2    die Messkammer der Detektionsvorrichtung von Figur 1 und zwei beispielhafte Strahlengänge für die beiden IR-Strahlen;
Figur 3    die Messkammer von Figur 2 mit einem alternativen Strahlengang für den ersten IR-Strahl.

Die erfindungsgemäße Vorrichtung vermag zu detektieren, ob in der Atemluft, die eine Testperson ausatmet, Alkohol enthalten ist oder nicht. Bevorzugt vermag die Vorrichtung weiterhin zu entscheiden, ob der Alkoholgehalt in der ausgeatmeten Atemluft oberhalb einer vorgegebenen Schranke liegt oder nicht, z.B. einer Nachweisschranke oder einer gesetzlich vorgegebenen Schranke. Optional vermag sie sogar den Alkoholgehalt in eine Atemprobe quantitativ zu bestimmen und auszugeben.

Die erfindungsgemäße Vorrichtung ist bevorzugt als von einem Menschen in einer Hand tragbare Vorrichtung ausgestaltet und lässt sich z.B. dafür verwenden, um Fahrzeugführer für Straßenfahrzeuge, Schienenfahrzeuge, Wasserfahrzeuge, Luftfahrzeuge oder auch Personen, die einem bestimmten Bereich betreten oder eine bestimmte Anlage überwachen wollen oder sollen, auf Alkohol zu kontrollieren.

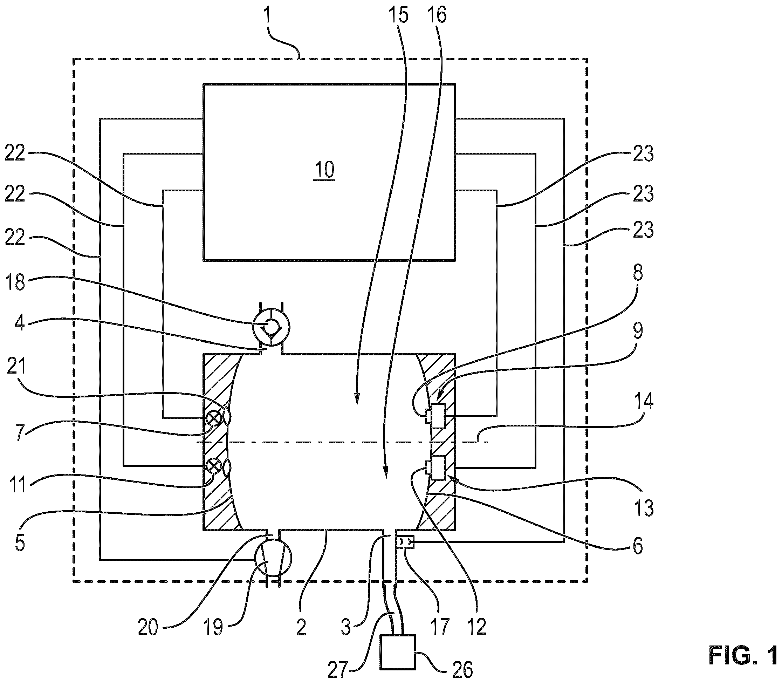

Figur 1 zeigt schematisch eine Ausgestaltung einer nicht beanspruchten Detektionsvorrichtung. Die Detektionsvorrichtung umfasst ein Gehäuse ("Küvettenkörper") 1, in dem eine Messkammer ("Küvette") 2 mit einem Einlass 3 und einem Auslass 4 angeordnet ist. Bevorzugt steht der Einlass 3 mit einer Eingabevorrichtung in Fluidkommunikation, beispielsweise mit einem Mundstück oder mit einem Trichter, in welches / welchen eine Person Atemluft hineinblasen kann. Gas, insbesondere ausgeatmete Atemluft, kann durch den Einlass 3 in die Messkammer 2 fließen und durch den Auslass 4 wieder aus der Messkammer 2 fließen. Figur 1 zeigt schematisch ein Mundstück 26, der über einen Schlauch 27 in Fluidverbindung mit dem Einlass 3 steht. In einer Ausgestaltung vermag ein nicht gezeigter Verschluss, z.B. eine Kappe, den Einlass 3 oder das Mundstück 26 oder den Schlauch 27 zu verschließen. Ein optionaler Durchflusssensor 17 misst den Volumenstrom, der durch den Einlass 3 in die Messkammer 2 fließt. Ein optionales Rückschlagventil 18 verhindert, dass Umgebungsluft durch den Auslass 4 in die Messkammer 2 einströmt und Messergebnisse verfälschen könnte. Hingegen ermöglicht das Rückschlagventil 18 es, das Gas durch den Auslass 4 aus der Messkammer 2 hinaus strömen kann.

Ein nicht gezeigter optionaler Filter am Einlass 3 verhindert, dass Wassertröpfchen, Staubteilchen oder sonstige störende Partikel in die Messkammer 2 gelangen. Bevorzugt hält eine nicht gezeigte Heizvorrichtung die Lufttemperatur in der Messkammer 2 auf mindestens 40° C. Dies reduziert das Risiko, dass Wasser auf einer Wand der Messkammer 2 kondensiert und das kondensierte Wasser Messergebnisse verfälschen oder einen Bestandteil beschädigen kann.

Eine nicht gezeigte Ausgabeeinheit gibt ein Untersuchungsergebnis in einer von einem Menschen wahrnehmbaren Form aus, insbesondere optisch und / oder akustisch. Zumindest wird ausgegeben, ob die Atemprobe Alkohol in einer Konzentration oberhalb einer vorgegebenen Schranke aufweist oder nicht, z.B. oberhalb einer Nachweisgrenze oder oberhalb einer gesetzlich vorgeschriebenen Schranke. Möglich ist auch, dass die Ausgabeeinheit einen Messwert betreffend die gemessene Alkoholkonzentration ausgibt. Beabstandet vom Einlass 3 ist ein optionaler Spülauslass 20 mit einer Pumpe 19 angeordnet. In einem Reinigungsmodus der Detektionsvorrichtung vermag die Pumpe 19 Fluid aus der Messkammer 2 herauszusaugen, insbesondere nachdem eine Person eine Atemprobe in die Messkammer 2 eingegeben hat und die Detektionsvorrichtung diese Atemprobe automatisch untersucht hat. Alkoholfreie Umgebungsluft kann durch den Einlass 3 in die Messkammer 2 strömen und ersetzt die untersuchte Gasprobe. Dadurch lässt sich die Detektionsvorrichtung für einen neuen Einsatz vorbereiten. Möglich ist auch, dass die Pumpe 19 eine alkoholhaltige Referenz-Gasprobe durch den Einlass 3 in die Messkammer 2 hinein fördert.

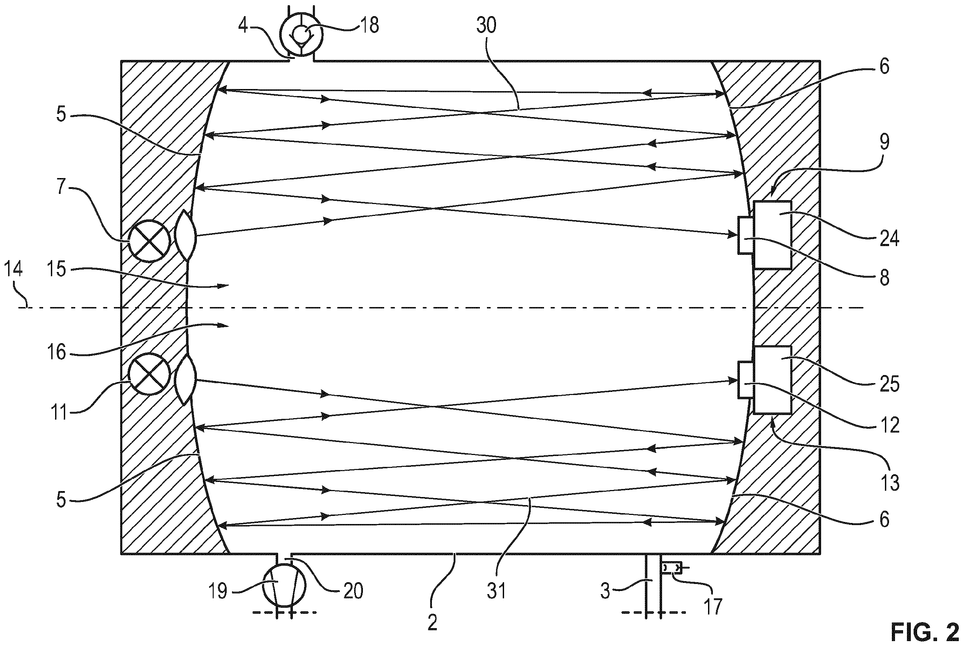

Figur 2 zeigt die Messkammer 2 im Detail. In diesem nicht beanspruchten Ausführungsbeispiel ist die Messkammer 2 symmetrisch bezüglich einer Symmetrieebene 14, welche senkrecht auf den Zeichenebenen von Figur 1 bis Figur 3 steht. Diese Symmetrieebene 14 unterteilt die Messkammer 2 in eine erste Hälfte 15 und in eine zweite Hälfte 16, die spiegelsymmetrisch zueinander sind. In der ersten Hälfte 15 ist somit eine erste Detektionseinheit angeordnet, welche die erste IR-Strahlungsquelle 7 und den ersten IR-Detektor 9 umfasst. In der zweiten Hälfte 16 ist eine zweite Detektionseinheit angeordnet, welche die zweite IR-Strahlungsquelle 9 und den zweiten IR-Detektor 13 umfasst.

Die Messkammer 2 kann auch unsymmetrisch aufgebaut sein. Zwei gegenüberliegende Seiten der Messkammer 2 werden erfindungsgemäß von jeweils einem Konkavspiegel 5 bzw. 6 gebildet, nämlich eine Seite von einem senderseitigen Konkavspiegel 5 und eine gegenüberliegende Seite von einem empfängerseitigen Konkavspiegel 6. Bevorzugt haben beiden Konkavspiegel 5, 6 dieselbe Brennweite. Bevorzugt liegen beide Brennpunkte idealerweise auf der optischen Achse 14.

In den senderseitigen Konkavspiegel 5 sind eine erste IR-Strahlungsquelle 7 und eine zweite IR-Strahlungsquelle 11 eingelassen, wobei die erste IR-Strahlungsquelle 7 zur ersten Hälfte 15 und die zweite IR-Strahlungsquelle 11 zur zweiten Hälfte 16 der Messkammer 2 gehört.

In den empfängerseitigen Konkavspiegel 6 sind ein erster IR-Detektor 9 und ein zweiter IR-Detektor 13 eingelassen, wobei der erste IR-Detektor 9 zur ersten Hälfte 15 und der zweite IR-Detektor 13 zur zweiten Hälfte 16 der Messkammer 2 gehört. Dies ist aber nicht beansprucht. Erfindungsgemäß sind die beiden IR-Detektoren (9,13) an den zwei gegenüberliegenden Wänden angeordnet. Der erste IR-Detektor 9 umfasst einen ersten Photosensor 24 und einen ersten Wellenlängen-Filter 8. Der zweite IR-Detektor 13 umfasst einen zweiten Photosensor 25 und einen zweiten Wellenlängen-Filter 12.

Bevorzugt hält ein nicht gezeigtes Heizelement das Innere der Messkammer 2 auf eine Temperatur von mindestens 40 °C, sodass weitgehend verhindert wird, dass Wasser auf einem Konkavspiegel 5 oder 6 oder einem Wellenlängen-Filter 8, 12 kondensiert.

Bevorzugt ist die Durchlässigkeit des ersten Wellenlängen-Filters 8 in einem ersten Wellenlängen-Bereich am größten, die Durchlässigkeit des zweiten Wellenlängen-Filters 12 in einem zweiten Wellenlängen-Bereich am größten. Die beiden Wellenlängen-Bereiche unterscheiden sich und umfassen einen gemeinsamen Teilbereich, in dem ein IR-Strahl durch Alkohol in der Atemluft ausreichend abgeschwächt wird. Beispielsweise hat der erste Wellenlängen-Bereich eine maximale Durchlässigkeit (Zentralwellenlänge) bei 9,6 $\mu$m, der zweite Wellenlängen-Bereich bei 9,2 $\mu$m.

Bevorzugt werden die beiden IR-Detektoren 9 und 13 so justiert oder kalibriert, dass sie - trotz unterschiedlicher Wellenlängen-Bereiche - die gleiche Empfindlichkeit für Ethanol, also für Alkohol in der Atemluft, aufweisen. Die Auswerteeinheit 10 wertet die Messwerte von den beiden IR-Detektoren 9 und 13 aber dergestalt aus, dass die beiden Detektionseinheiten unterschiedliche Empfindlichkeiten für andere Substanzen aufweisen, die in der ausgeatmeten Atemluft sein können, beispielsweise Kohlendioxid, Isopropanol, Methanol oder Aceton. Auf diese Weise lässt sich Ethanol besonders gut von einer anderen Substanz, die sich in einer eingegebenen Atemprobe befinden kann, unterscheiden.

In einer bevorzugten Ausgestaltung werden vorab zwei Kalibrierungsfaktoren k1 und k2, beispielsweise Gewichtsfaktoren, für die beiden Detektionseinheiten mit den beiden IR-Detektoren 9 und 13 ermittelt und für eine Justierung der Detektionsvorrichtung verwendet. Diese Justierung lässt sich regelmäßig wiederholen. In einer Realisierung wird die Justierung wie folgt durchgeführt:

- Die Messkammer 2 wird mit Umgebungsluft gefüllt, die frei von Alkohol ist, beispielsweise indem die Pumpe 19 die Messkammer 2 leersaugt und alkoholfreie Umgebungsluft durch den Einlass 3 in die Messkammer 2 einströmt, um den erzeugten Unterdruck auszugleichen.
- Jede IR-Strahlungsquelle 7, 11 sendet jeweils einen IR-Strahl in die Messkammer 2. Jeder IR-Detektor 9 und 13 misst jeweils einen Referenz-Null-Messwert I0_ref(1) bzw. I0_ref(2), während die Messkammer 2 frei von Alkohol ist.
- Die Messkammer 2 wird mit einer Referenz-Gasprobe gefüllt, welche Alkohol enthält, bevorzugt in einer Konzentration, die typisch für ausgeatmete Atemluft nach dem Konsum von Alkohol ist.
- Jede IR-Strahlungsquelle 7, 11 sendet erneut jeweils einen IR-Strahl in die Messkammer 2. Jeder IR-Detektor 9 und 13 misst jeweils einen Referenz-Messwert I1_ref(1) bzw. I1_ref(2), während die Messkammer 2 mit der alkoholhaltige Referenz-Gasprobe gefüllt ist.
- Die beiden Kalibrierungsfaktoren k1 und k2 werden so gewählt, dass sie dieselbe Sensitivität der beiden Detektionseinheiten mit den beiden IR-Detektoren 9 und 13 bewirken.

Dies wird beispielsweise wie folgt durchgeführt:
In den beiden Gleichungen

$$C\_ref(1) = k1 * [I0\_ref(1) - I1\_ref(1)] / I0\_ref(1)$$

und

$$C\_ref(2) = k2 * [I0\_ref(2) - I1\_ref(2)] / I0\_ref(2)$$

werden die beiden Kalibrierungsfaktoren k1 und k2 so gewählt, dass C_ref(1) = C_ref(2) gilt.

Möglich ist, diese Kalibrierung regelmäßig zu wiederholen, um auf diese Weise allmähliche Veränderungen eines Bestandteils der Detektionsvorrichtung zu kompensieren.

Möglich ist auch, dass die Justierung durchgeführt wird, indem die beiden Wellenlängen-Filter 8 und 12 entsprechend eingestellt werden. Ein Beispiel hierfür wird nachfolgend beschrieben.

In einer Realisierung lässt sich jeder Wellenlängen-Filter 8 und 12 wahlweise in einem ersten Modus oder in einem zweiten Modus betreiben. Bei Betrieb im ersten Modus ist die Durchlässigkeit im ersten Wellenlängen-Bereich am größten, bei Betrieb im zweiten Modus die Durchlässigkeit im zweiten Wellenlängen-Bereich. Diese Ausgestaltung ermöglicht es, bei der Untersuchung einer Atemprobe zunächst den ersten Wellenlängen-Filter 8 im ersten Modus und dem zweiten Wellenlängen-Filter 12 im zweiten Modus zu betreiben und anschließend umgekehrt den ersten Wellenlängen-Filter 8 im zweiten Modus und dem zweiten Wellenlängen-Filter 12 im ersten Modus. Auf diese Weise wird die Auswirkung einer nicht idealen Justierung wenigstens teilweise kompensiert. Außerdem wird ermöglicht, dass jeder IR-Detektor jeweils zwei Messwerte liefert, während die Messkammer 2 mit einer zu untersuchenden Atemprobe gefüllt ist.

Figur 2 veranschaulicht ein Beispiel für den Strahlengang 30 eines IR-Strahls, den die erste IR-Strahlungsquelle 7 in die Messkammer 2 hinein emittiert hat, der mehrfach von beiden Konkavspiegeln 5 und 6 reflektiert wird und der anschließend auf den ersten IR-Detektor 9 auftrifft. Weiterhin veranschaulicht

Figur 2 den Strahlengang 31 eines IR-Strahls, den die zweite IR-Strahlungsquelle 11 in die Messkammer 2 hinein emittiert hat. Im gezeigten Beispiel wird jeder IR-Strahl nach der Emission jeweils siebenmal reflektiert, bevor er auf einen IR-Detektor 9 oder 13 auftritt.

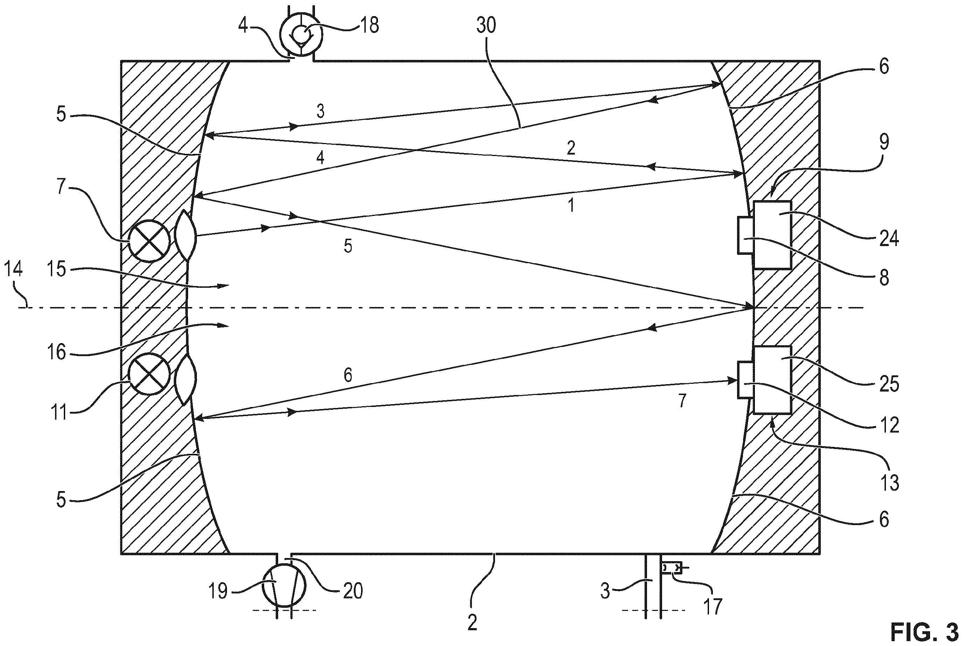

Im Beispiel von Figur 2 nutzt der erste Strahlengang 30 nur die erste Hälfte 15, und der zweite Strahlengang 31 nutzt nur die zweite Hälfte 16. Figur 3 zeigt eine Abwandlung, bei welcher der erste Strahlengang 30 und der in Figur 3 nicht gezeigte zweite Strahlengang 31 jeweils fast die gesamte Messkammer 2 ausnutzen. Der zum ersten Strahlengang 30 spiegelsymmetrische zweite Strahlengang 31 wird in Figur 3 nicht gezeigt.

Bevorzugt beträgt die maximale Abmessung der Messkammer 2 parallel zur Symmetrieebene 14 10 cm. Weil jeder emittierte IR-Strahl mehrmals an den beiden Konkavspiegeln 5 und 6 reflektiert wird, kann jeder Strahlengang 30 und 31 eine Länge von 40 cm oder mehr aufweisen, die sogenannte Absorptionslänge. Die Absorptionslänge beträgt bei sechsfacher Reflexion das Siebenfache des Abstands zwischen den beiden Konkavspiegel 5 und 6. Weiterhin wird ermöglicht, dass der Einfallswinkel eines IR-Strahls auf einen IR-Detektor 9 oder 13 maximal 30 Grad beträgt, was zu einer noch ausreichenden Apertur führt.

Bevorzugt ist die Messkammer 2 mit den beiden Konkavspiegeln 5 und 6 als Herriott-Zelle ausgestaltet. Der Abstand d zwischen den beiden Konkavspiegeln 5 und 6 sowie die bevorzugt übereinstimmende Brennweite f der beiden Konkavspiegel 5 und 6 sind so ausgelegt, dass die beiden IR-Strahlungsquellen 7 und 11 idealerweise scharf und im Maßstab 1:1 auf die beiden IR-Detektoren 9 und 13 abgebildet werden. Beispielsweise werden eine bestimmte Brennweite f der beiden Konkavspiegel 5 und 6 und für eine scharfe Abbildung ein bestimmtes Verhältnis f/d vorgegeben. Hieraus resultiert ein Abstand d zwischen den beiden Konkavspiegeln 5 und 6. Aus der Anzahl, wie oft ein emittierter IR-Strahl reflektiert wird, resultiert die erzielte Absorptionslänge. Eine leichte Fehlstellung eines Konkavspiegel 5 oder 6 beeinträchtigt daher die Messergebnisse nur gering.

Eine optionale erste verstellbare Umlenkvorrichtung 21 vermag einen IR-Strahl, den die erste IR-Strahlungsquelle 7 emittiert hat, wahlweise auf den ersten IR-Detektor 9 oder den zweiten IR-Detektor 13 zu lenken oder wenigstens die Richtung des IR-Strahl zu verändern. Beispielsweise vermag die Umlenkvorrichtung 21 die erste IR-Strahlungsquelle 7 zu drehen. Eine nicht gezeigte optionale zweite Umlenkvorrichtung vermag einen IR-Strahl, den die zweite IR-Strahlungsquelle 11 emittiert hat, wahlweise auf den ersten IR-Detektor 9 oder den zweiten IR-Detektor 13 zu lenken. Diese Ausgestaltung ermöglicht es, die Detektionsvorrichtung auch dann mit zwei IR-Detektoren 9 und 13 zu betreiben, wenn eine der beiden IR-Strahlungsquelle 7 oder 11 ausgefallen ist. Außerdem lässt sich eine leicht fehlerhafte Position einer IR-Strahlungsquelle 7, 11 oder eines IR-Detektors 9, 13 korrigieren.

Eine datenverarbeitende Auswerteeinheit 10 vermag über Steuerleitungen 22 Steuerbefehle an die erste IR-Strahlungsquelle 7, die zweite IR-Strahlungsquelle 11 und die Pumpe 19 und optional an eine Umlenkeinheit 21 zu übermitteln. Der erste Photosensor 24 und der zweite Photosensor 25 erzeugen unabhängig voneinander jeweils mindestens einen Messwert, wobei diese Messwerte jeweils von der Intensität eines auf den Photosensor 24 bzw. 25 auftreffenden IR-Strahls 30, 31 abhängen. Über Sensorleitungen 23 werden Messwerte von dem ersten Photosensor 24, dem zweiten Photosensor 25 sowie dem Durchflusssensor 17 an die Auswerteeinheit 10 übermittelt.

Die Detektionsvorrichtung des Ausführungsbeispiels umfasst weiterhin eine nicht gezeigte eigene Energieversorgung für elektrische Energie, beispielsweise einen Satz von wiederaufladbaren Batterien, welche die Detektionsvorrichtung unabhängig von einer stationären Versorgung machen.

Bevorzugt werden die folgenden Verfahrensschritte durchgeführt, um eine Messung auf Alkohol in der Atemluft eine Person durchzuführen:

Die Auswerteeinheit 10 aktiviert über eine Steuerleitung 22 die Pumpe 19.

Die aktivierte Pumpe 19 saugt Gas aus der Messkammer 2. Das abgesogene Gas wird durch Umgebungsluft ersetzt, welches durch den Einlass 3 in die Messkammer 2 fließt, um den erzeugten Unterdruck auszugleichen. Auf diese Weise wird Gas, welches von einer vorherigen Atemprobe stammt, durch eine alkoholfreie Referenz-Gasprobe ersetzt. Daher kann eine vorherige Gasprobe nicht das Ergebnis verfälschen.

Die Auswerteeinheit 10 wertet einen Messwert von dem Durchflusssensor 17 aus und detektiert das Ereignis, dass das Volumen der in die Messkammer 2 eingesogenen Umgebungsluft mindestens so groß wie das Volumen der Messkammer 2 ist. Die Auswerteeinheit 10 schaltet die Pumpe 19 wieder ab, sobald dieses Ereignis detektiert ist. Nunmehr befindet sich in der Messkammer 2 eine alkoholfreie Gasprobe.

In einer Ausgestaltung werden ein erster Null-Messwert 10(1) und ein zweiter Null-Messwert I0(2) aus einem Datenspeicher eingelesen. In einer anderen Ausgestaltung emittieren beide IR-Strahlungsquellen 7 und 11 jeweils einen IR-Strahl, und die beiden IR-Detektoren 9 und 13 messen den ersten Null-Messwert 10(1) bzw. den zweiten Null-Messwert I0(2). Die beiden Null-Messwerte 10(1) und I0(2) der beiden IR-Detektoren 9 und 13 liegen vor, wenn das Gas in der Messkammer 2 keinen Alkohol aufweist. Die Ausgestaltung, vor jeder Alkoholprobe erneut die beiden Null-Messwerte 10(1) und I0(2) zu messen, hat insbesondere folgenden Vorteil: Eine allmähliche Veränderung eines Bestandteils der Detektionsvorrichtung wird automatisch kompensiert. Insbesondere wird eine allmähliche Veränderung einer IR-Strahlungsquelle 7, 11 oder eines IR-Detektors 9, 13 oder der Spannungsquelle kompensiert. Ausreichend ist, dass die beiden IR-Strahlungsquellen 7, 11 und die beiden IR-Detektoren 9, 13 während einer einzigen Alkoholmessung praktisch unverändert bleiben.

Eine Atemprobe mit zu untersuchender Atemluft wird durch den Einlass 3 in die Messkammer 2 verbracht. Beispielsweise bläst eine Person in das Mundstück 26, und die eingegebene Luft fließt durch den Schlauch 27 und den Einlass 3 in die Messkammer 2.

Die erste IR-Strahlungsquelle 7 emittiert einen IR-Strahl in die Messkammer 2. Der emittierte IR-Strahl durchdringt entlang des ersten Strahlenweges 30 die Messkammer 2 und trifft auf den ersten IR-Detektor 9.

Die zweite IR-Strahlungsquelle 11 emittiert ebenfalls einen IR-Strahl in die Messkammer 2. Der emittierte IR-Strahl durchdringt entlang des zweiten Strahlenweges 31 die Messkammer 2 und trifft auf den zweiten IR-Detektor 13.

Möglich ist, dass die bei den IR-Strahlungsquellen 7 und 11 zeitlich überlappend oder sogar gleichzeitig jeweils einen IR-Strahl emittieren. Möglich ist auch, dass die beiden IR-Strahlungsquellen 7 und 11 nacheinander jeweils einen IR-Strahl emittieren.

Jeder IR-Detektor 9, 13 liefert jeweils ein Messwert I1(1) bzw. I1(2) für die Lichtintensität, die am Photosensor 24 bzw. 25 auftritt, während die Atemprobe sich in der Messkammer 2 befindet.

Die Auswerteeinheit 10 berechnet zwei Signale C(1) und C(2), die jeweils ein Maß für die Konzentration von Ethanol in der Atemprobe sind, abhängig von den beiden Null-Messwerten I0(1), I0(2) und den beiden Messwerten I1(1), I1(2), beispielsweise gemäß den folgenden beiden Formeln

$$C(1) = k1 * [I0(1) - I1(1)] / I0(1)$$

und

$$C(2) = k2 * [I0(2) - I1(2)] / I0(2).$$

Die Kalibrierungsfaktoren k1 und k2 sind vorgegeben oder wurden vorab bestimmt und sind in einem Datenspeicher der Detektionsvorrichtung abgespeichert. Die Kalibrierungsfaktoren k1 und k2 wurden beispielsweise so wie oben beschrieben festgelegt.

Falls die Atemprobe keine störenden Substanzen enthält, so stimmen die beiden Signale C(1) und C(2) idealerweise überein.

Bevorzugt prüft die Auswerteeinheit 10, ob die absolute oder prozentuale Abweichung zwischen den beiden Signalen C(1) und C(2) unterhalb einer vorgegebenen Schranke liegt. Falls dies der Fall ist, so wird das Messergebnis als korrekt angesehen. Die Schranke wird einerseits so niedrig vorgegeben, dass das Ergebnis bei einer Abweichung zwischen den beiden Signalen C(1) und C(2) unterhalb der Schranke vertrauenswürdig ist, auch wenn andere Substanzen und auch störende Strahlung in die Messkammer 2 eingedrungen sind. Andererseits wird die Schranke so hoch vorgegeben, das unvermeidliche Abweichungen zwischen den Messergebnissen der beiden IR-Detektoren 9 und 13 nicht dazu führen, dass ein korrektes Ergebnis verworfen wird.

Durch diese Ausgestaltung lässt sich insbesondere erkennen, dass eine IR-Strahlungsquelle 7, 11 oder ein erster IR-Detektor 9, 13 defekt ist oder ein Strahlengang 30, 31 blockiert ist.

**Bezugszeichenliste**

| 1 | Gehäuse (Küvettenkörper), welches die Messkammer 2 und die Auswerteeinheit 10 aufnimmt |
|---|---|

(fortgesetzt)

| | |
|---|---|
| 2 | gasdichte Messkammer zur Aufnahme einer Gasprobe, umfasst den Einlass 3, den Auslass 4 und den Spülauslass 20, im Gehäuse 1 angeordnet |
| 3 | Einlass für Atemluft, mit dem Mundstück 26 verbunden |
| 4 | Auslass für Atemluft |
| 5 | senderseitiger Konkavspiegel, bildet eine Wand der Messkammer 2, nimmt die beiden IR-Strahlungsquellen 7 und 11 auf |
| 6 | empfängerseitiger Konkavspiegel, bildet eine Wand der Messkammer 2, nimmt die beiden IR-Detektoren 9 und 13 auf |
| 7 | erste IR-Strahlungsquelle, im senderseitigen Konkavspiegel 5 angeordnet |
| 8 | erster Wellenlängen-Filter, gehört zum ersten IR-Detektor 9 |
| 9 | erster IR-Detektor, im empfängerseitigen Konkavspiegel 6 angeordnet, umfasst den ersten Wellenlängen-Filter 8 und den ersten Photosensor 24 |
| 10 | signalverarbeitende Auswerteeinheit, steuert die beiden IR-Strahlungsquellen 7 und 11 an, empfängt Messwerte von den beiden Photosensoren 24, 25 und verarbeitet diese |
| 11 | zweite IR-Strahlungsquelle, im senderseitigen Konkavspiegel 5 angeordnet |
| 12 | zweiter Wellenlängen-Filter, gehört zum zweiten IR-Detektor 13 |
| 13 | zweiter IR-Detektor, im empfängerseitigen Konkavspiegel 6 angeordnet, umfasst den zweiten Wellenlängen-Filter 12 und den zweiten Photosensor 25 |
| 14 | Symmetrieebene und optische Achse der Messkammer 2, unterteilt die Messkammer 2 in die beiden Hälften 15 und 16 |
| 15 | erste Hälfte der Messkammer 2, nimmt die erste IR-Strahlungsquelle 7 und den ersten IR-Detektor 9 auf |
| 16 | zweite Hälfte der Messkammer 2, nimmt die zweite IR-Strahlungsquelle 11 und den zweiten IR-Detektor 13 auf |
| 17 | Durchflusssensor am Einlass 3 |
| 18 | Rückschlagventil am Auslass 4 |
| 19 | ansteuerbare Pumpe am Spülauslass 20 |
| 20 | Spülauslass 20 |
| 21 | Erste Umlenkvorrichtung, vermag einen IR-Strahl von der ersten IR-Strahlungsquelle 7 zum zweiten IR-Detektor 13 umzulenken, vermag beispielsweise die erste IR-Strahlungsquelle 7 zu drehen |
| 22 | Steuerleitung von der Auswerteeinheit 10 |
| 23 | Sensorleitung zu der Auswerteeinheit 10 |
| 24 | erster Photosensor, gehört zum ersten IR-Detektor 9 |
| 25 | zweiter Photosensor, gehört zum zweiten IR-Detektor 13 |
| 26 | Mundstück, steht über den Schlauch 27 in Fluidverbindung mit dem Einlass 3 |
| 27 | Schlauch, stellt eine Fluidverbindung zwischen dem Mundstück 26 und dem Einlass 3 hier |
| 30 | Strahlengang eines IR-Strahls in der Messkammer 2 von der ersten IR-Strahlungsquelle 7 zum ersten IR-Detektor 9 |
| 31 | Strahlengang eines IR-Strahls in der Messkammer 2 von der zweiten IR-Strahlungsquelle 11 zum zweiten IR-Detektor 13 |

**Patentansprüche**

1. Vorrichtung zur Detektion von Alkohol in einer Gasprobe, insbesondere in ausgeatmeter Atemluft,

wobei die Vorrichtung

- eine Messkammer (2) zum Aufnehmen einer Gasprobe,
- eine erste IR-Strahlungsquelle (7),
- eine zweite IR-Strahlungsquelle (11),
- einen ersten IR-Detektor (9),
- einen zweiten IR-Detektor (13) und
- eine signalverarbeitende Auswerteeinheit (10) umfasst,

wobei an zwei gegenüberliegenden Wänden der Messkammer (2) jeweils ein Spiegel (5, 6) angeordnet ist,
wobei die beiden IR-Detektoren (9, 13) an den zwei gegenüberliegenden Wänden (5, 6) der Messkammer (2) angeordnet sind,
wobei jede der beiden IR-Strahlungsquellen (7, 11) dazu ausgestaltet ist, jeweils einen IR-Strahl in die Messkammer (2) zu emittieren,
wobei jeder der beiden IR-Detektoren (9, 13) dazu ausgestaltet ist, abhängig von einem auftreffenden IR-Strahl mindestens einen Messwert zu erzeugen,
wobei die Vorrichtung so ausgestaltet ist, dass

- ein IR-Strahl von der ersten IR-Strahlungsquelle (7) die Messkammer (2) durchdringt und auf den ersten IR-Detektor (9) trifft und
- ein IR-Strahl von der zweiten IR-Strahlungsquelle (11) die Messkammer (2) durchdringt und auf den zweiten IR-Detektor (13) trifft,

wobei die Vorrichtung so ausgestaltet ist, dass

- ein von der ersten IR-Strahlungsquelle (7) emittierter IR-Strahl von mindestens einem Spiegel (5, 6) mindestens einmal, bevorzugt mehrmals, reflektiert wird, bevor er auf den ersten IR-Detektor (9) auftrifft, und
- ein von der zweiten IR-Strahlungsquelle (11) emittierter IR-Strahl von mindestens einem Spiegel (5, 6) mindestens einmal, bevorzugt mehrmals, reflektiert wird, bevor er auf den zweiten IR-Detektor (13) auftrifft, und

wobei die Auswerteeinheit (10) dazu ausgestaltet ist,

- automatisch zu entscheiden, ob eine Gasprobe in der Messkammer (2) Alkohol enthält oder nicht, und
- diese Entscheidung unter Verwendung von Messwerten zu fällen, welche die beiden IR-Detektoren (9, 13) erzeugt haben, während sich eine zu untersuchende Gasprobe in der Messkammer (2) befindet.

2. Vorrichtung nach Anspruch 1,
   **dadurch gekennzeichnet, dass**
   die beiden IR-Detektoren (9, 13) dazu ausgestaltet sind, unabhängig voneinander zu arbeiten.

3. Vorrichtung nach einem der vorhergehenden Ansprüche,

   **dadurch gekennzeichnet, dass**
   der erste IR-Detektor (9) einen ersten Wellenlängen-Filter (8) und einen ersten Photosensor (24) umfasst und der zweite IR-Detektor (13) einen zweiten Wellenlängen-Filter (12) und einem zweiten Photosensor (25) umfasst,
   wobei der erste Wellenlängen-Filter (8) dazu ausgestaltet ist, einen auftreffenden IR-Strahl so zu filtern, dass ein IR-Teilstrahl in einem ersten Wellenlängen-Bereich den ersten Wellenlängen-Filter (8) passiert und auf den ersten Photosensor (24) auftrifft,
   wobei der zweite Wellenlängen-Filter (12) dazu ausgestaltet ist, einen auftreffenden IR-Strahl so zu filtern, dass ein IR-Teilstrahl in einem zweiten Wellenlängen-Bereich den zweiten Wellenlängen-Filter (12) passiert und auf den zweiten Photosensor (25) auftrifft, und
   wobei die beiden Wellenlängen-Bereiche voneinander verschieden sind.

4. Vorrichtung nach Anspruch 3,

   **dadurch gekennzeichnet, dass**
   mindestens einer der beiden Wellenlängen-Filter (8, 12) wahlweise in einem ersten Modus oder in einem zweiten Modus betreibbar ist,
wobei der Wellenlängen-Filter dazu ausgestaltet ist,

- im ersten Modus einen auftreffenden IR-Strahl so zu filtern, dass ein IR-Teilstrahl in dem ersten Wellenlängen-Bereich den Wellenlängen-Filter (8, 12) passiert, und
- im zweiten Modus einen auftreffenden IR-Strahl so zu filtern, dass ein IR-Teilstrahl in dem zweiten Wellenlängen-Bereich den Wellenlängen-Filter (8, 12) passiert.

**5.** Vorrichtung nach einem der vorhergehenden Ansprüche,

**dadurch gekennzeichnet, dass**
die Auswerteeinheit (10) dazu ausgestaltet ist,

- abhängig von einem Messwert des ersten IR-Detektors (9) ein erstes Signal zu erzeugen und
- abhängig von einem Messwert des zweiten IR-Detektors (13) ein zweites Signal zu erzeugen,

wobei beide Signale jeweils ein Maß für den Gehalt an Ethanol in einer Gasprobe in der Messkammer (2) sind und wobei die Auswerteeinheit (10) weiterhin dazu ausgestaltet ist, bei der Entscheidung, ob die Gasprobe in der Messkammer (2) Alkohol enthält oder nicht, die beiden Signale miteinander zu vergleichen.

**6.** Vorrichtung nach Anspruch 5,

**dadurch gekennzeichnet, dass**
die Detektionsvorrichtung so ausgestaltet ist, dass die beiden Signale

- die gleiche Empfindlichkeit für Ethanol,
- aber unterschiedliche Empfindlichkeiten für mindestens eine andere Substanz, die in einer Gasprobe in der Messkammer (2) auftreten kann, aufweisen.

**7.** Vorrichtung nach einem der vorhergehenden Ansprüche,

**dadurch gekennzeichnet, dass**
die beiden IR-Strahlungsquellen (7, 11) an zwei gegenüberliegenden Wänden (5, 6) der Messkammer (2) angeordnet sind oder
die beiden IR-Strahlungsquellen (7, 11) voneinander beabstandet an derselben Wand (5) der Messkammer (2) angeordnet sind.

**8.** Vorrichtung nach einem der vorhergehenden Ansprüche,

**dadurch gekennzeichnet, dass**
beide Spiegel (5, 6) als Konkavspiegel ausgebildet sind,
wobei der Abstand zwischen den beiden Spiegeln (5, 6) und die Brennweiten der beiden Konkavspiegel (5, 6) so ausgestaltet sind, dass

- die erste IR-Strahlungsquelle (7) scharf auf den ersten IR-Detektor (9) abgebildet wird und
- die zweite IR-Strahlungsquelle (11) scharf auf den zweiten IR-Detektor (13) abgebildet wird.

**9.** Vorrichtung nach einen der vorhergehenden Ansprüche,

**dadurch gekennzeichnet, dass**
dieselben beiden einander gegenüberliegenden Wände (5, 6) der Messkammer (2)

- die beiden IR-Strahlungsquellen (7, 11),
- die beiden IR-Detektoren (9, 13) und
- die beiden Spiegel (5, 6) aufnehmen.

**10.** Vorrichtung nach einem der vorhergehenden Ansprüche,

**dadurch gekennzeichnet, dass**
die Vorrichtung dazu ausgestaltet ist, die Messkammer (2) mit einer Gasprobe zu füllen, die frei von Alkohol ist, und die Schritte auszulösen, dass

- jede IR-Strahlungsquelle (7, 11) jeweils einen IR-Strahl in die mit der alkoholfreien Gasprobe gefüllten Messkammer (2) emittiert und
- jeder IR-Detektor (9, 13) abhängig von einem bei der alkoholfreien Gasprobe auftreffenden IR-Strahl jeweils einen Null-Messwert erzeugt,

wobei die Auswerteeinheit (10) dazu ausgestaltet ist, die Entscheidung über eine auf Alkohol zu untersuchende Gasprobe in der Messkammer (2)

- in Abhängigkeit von Messwerten, welche die beiden IR-Detektoren (9, 13) erzeugt haben, während die Messkammer (2) mit der zu untersuchenden Gasprobe gefüllt ist, und
- zusätzlich in Abhängigkeit von den beiden Null-Messwerten zu treffen.

**11.** Verfahren zum Detektieren von Alkohol in einer Gasprobe, insbesondere in ausgeatmeter Atemluft,

unter Verwendung einer Detektionsvorrichtung, die

- eine Messkammer (2) zur Aufnahme einer Gasprobe,
- eine erste IR-Strahlungsquelle (7),
- eine zweite IR-Strahlungsquelle (11),
- einen ersten IR-Detektor (9) und
- einen zweiten IR-Detektor (13) umfasst,

wobei an zwei gegenüberliegenden Wänden der Messkammer (2) jeweils ein Spiegel (5, 6) angeordnet ist,
wobei die beiden IR-Detektoren (9, 13) an den zwei gegenüberliegenden Wänden (5, 6) der Messkammer (2) angeordnet sind,
wobei das Verfahren die Schritte umfasst, dass

- eine auf Alkohol zu prüfende Gasprobe in die Messkammer (2) verbracht wird,
- jede der beiden IR-Strahlungsquellen (7, 11) jeweils einen IR-Strahl in die Messkammer (2) emittiert, während sich die zu prüfende Gasprobe in der Messkammer (2) befindet,
- ein IR-Strahl von der ersten IR-Strahlungsquelle (7) die Messkammer (2) durchdringt und auf den ersten IR-Detektor (9) trifft,
- ein IR-Strahl von der zweiten IR-Strahlungsquelle (11) die Messkammer (2) durchdringt und auf den zweiten IR-Detektor (13) trifft,
- jeder IR-Detektor (9, 13) abhängig von einem auftreffenden IR-Strahl jeweils mindestens einen Messwert erzeugt, während sich die zu prüfende Gasprobe in der Messkammer (2) befindet, und
- automatisch entschieden wird, ob die Gasprobe in der Messkammer (2) Alkohol enthält oder nicht, und
- wobei die Entscheidung unter Verwendung mindestens der beiden Messwerte von den beiden IR-Detektoren (9, 13) gefällt wird und

wobei

- ein von der ersten IR-Strahlungsquelle (7) emittierter IR-Strahl von mindestens einem Spiegel (5, 6) mindestens einmal, bevorzugt mehrmals, reflektiert wird,
bevor er auf den ersten IR-Detektor (9) auftrifft, und
- ein von der zweiten IR-Strahlungsquelle (11) emittierter IR-Strahl von mindestens einem Spiegel (5, 6) mindestens einmal, bevorzugt mehrmals, reflektiert wird,
bevor er auf den zweiten IR-Detektor (13) auftrifft.

**12.** Verfahren nach Anspruch 11,

**dadurch gekennzeichnet, dass**
das Verfahren die weiteren Schritte umfasst, dass

- die Messkammer (2) mit einer Gasprobe gefüllt wird, die frei von Alkohol ist,
- jede IR-Strahlungsquelle (7, 11) jeweils einen IR-Strahl in die Messkammer (2) emittiert, während die Messkammer (2) mit der alkoholfreien Gasprobe gefüllt ist, und
- jeder IR-Detektor (9, 13) jeweils abhängig von einem auftreffenden IR-Strahl jeweils mindestens einen Null-Messwert erzeugt, während die Messkammer (2) mit der alkoholfreien Gasprobe gefüllt ist, und

der Schritt, automatisch zu entscheiden, ob die zu untersuchende Gasprobe in der Messkammer (2) Alkohol enthält oder nicht,
zusätzlich unter Verwendung der beiden Null-Messwerte durchgeführt wird.

**13.** Verfahren nach Anspruch 12,

**dadurch gekennzeichnet, dass**
das Verfahren nacheinander für mindestens zwei verschiedene auf Alkohol zu untersuchenden Gasproben durchgeführt wird,
wobei vor oder nach den Schritten,

- die jeweilige zu untersuchende Gasprobe in die Messkammer (2) zu verbringen und
- die IR-Strahlen zu emittieren und die Messwerte zu erzeugen, während die zu untersuchende Gasprobe in der Messkammer (2) ist,

die Schritte durchgeführt werden, dass

- die alkoholfreie Gasprobe in die Messkammer (2) verbracht wird und
- die IR-Strahlen emittiert werden und die Null-Messwerte erzeugt werden, während die alkoholfreie Gasprobe in der Messkammer (2) ist,

wobei für jede Gasprobe die Entscheidung, ob die Gasprobe Alkohol enthält oder nicht, unter Verwendung

- von Messwerten, die erzeugt wurden, während diese Gasprobe in der Messkammer (2) ist, und
- von zwei Null-Messwerten von den beiden IR-Detektoren (9, 13) gefällt wird.

**14.** Verfahren nach einem der Ansprüche 11 bis 13,

**dadurch gekennzeichnet, dass**
der Schritt zu entscheiden, ob die Gasprobe in der Messkammer (2) Alkohol enthält oder nicht,
unter Verwendung von Messwerten der beiden IR-Detektoren (9, 13) und zusätzlich unter Verwendung von jeweils einem Kalibrierungsfaktor für die beiden IR-Detektoren (9, 13) durchgeführt wird,
wobei die beiden Kalibrierungsfaktoren automatisch generiert werden, bevor die auf Alkohol zu prüfende Gasprobe in die Messkammer (2) verbracht wird, und wobei die Generierung der beiden Kalibrierungsfaktoren die Schritte umfasst, dass

- die Messkammer (2) mit einer Gasprobe gefüllt wird, welche Ethanol enthält, und
- die beiden Kalibrierungsfaktoren dergestalt generiert werden, dass bei dieser alkoholhaltigen Gasprobe die beiden IR-Detektoren (9, 13) die gleiche Empfindlichkeit auf Ethanol aufweisen.

**Claims**

**1.** Device for detecting alcohol in a gas sample, in particular in exhaled respiratory air,

wherein the device comprises

- a measuring chamber (2) for receiving a gas sample,
- a first IR radiation source (7),
- a second IR radiation source (11),
- a first IR detector (9),
- a second IR detector (13) and

- a signal-processing evaluation unit (10),

wherein a respective mirror (5, 6) is arranged on each of two opposite walls of the measuring chamber (2),
wherein the two IR detectors (9, 13) are arranged on the two opposing walls (5, 6) of the measuring chamber (2),
wherein each of the two IR radiation sources (7, 11) is designed to emit a respective IR beam into the measuring chamber (2),
wherein each of the two IR detectors (9, 13) is designed to generate at least one measurement value on the basis of an incident IR beam,
wherein the device is designed such that

- an IR beam from the first IR radiation source (7) passes through the measuring chamber (2) and strikes the first IR detector (9) and
- an IR beam from the second IR radiation source (11) passes through the measuring chamber (2) and strikes the second IR detector (13),

wherein the device is designed such that

- an IR beam emitted by the first IR radiation source (7) is reflected at least once, preferably multiple times, by at least one mirror (5, 6), before said IR beam strikes the first IR detector (9) and
- an IR beam emitted by the second IR radiation source (11) is reflected at least once, preferably multiple times, by at least one mirror (5, 6), before said IR beam strikes the second IR detector (13), and

wherein the evaluation unit (10) is designed

- to automatically decide whether or not a gas sample in the measuring chamber (2) contains alcohol and
- to make this decision using measurement values which the two IR detectors (9, 13) generated while a gas sample to be examined is situated in the measuring chamber (2).

2. Device according to Claim 1,
   **characterized in that**
   the two IR detectors (9, 13) are designed to operate independently of one another.

3. Device according to either of the preceding claims,

   **characterized in that**
   the first IR detector (9) comprises a first wavelength filter (8) and a first photosensor (24) and
   the second IR detector (13) comprises a second wavelength filter (12) and a second photosensor (25),
   wherein the first wavelength filter (8) is designed to filter an incident IR beam in such a way that an IR partial beam in a first wavelength range passes the first wavelength filter (8) and strikes the first photosensor (24),
   wherein the second wavelength filter (12) is designed to filter an incident IR beam in such a way that an IR partial beam in a second wavelength range passes the second wavelength filter (12) and strikes the second photosensor (25), and
   wherein the two wavelength ranges differ from one another.

4. Device according to Claim 3,

   **characterized in that**
   at least one of the two wavelength filters (8, 12) is selectively operable in a first mode or in a second mode, wherein the wavelength filter is designed

   - to filter an incident IR beam in such a way in the first mode that an IR partial beam in the first wavelength range passes the wavelength filter (8, 12) and
   - to filter an incident IR beam in such a way in the second mode that an IR partial beam in the second wavelength range passes the wavelength filter (8, 12).

5. Device according to any one of the preceding claims,

   **characterized in that**

the evaluation unit (10) is designed

- to generate a first signal on the basis of a measurement value of the first IR detector (9) and
- to generate a second signal on the basis of a measurement value of the second IR detector (13),

wherein both signals respectively are a measure for the ethanol content in a gas sample in the measuring chamber (2) and
wherein the evaluation unit (10) is furthermore designed to compare the two signals to one another within the scope of the decision as to whether or not the gas sample in the measuring chamber (2) contains alcohol.

6. Device according to Claim 5,
**characterized in that**
the detection device is designed such that the two signals

- have the same sensitivity to ethanol
- but different sensitivities to at least one other substance that may occur in a gas sample in the measuring chamber (2).

7. Device according to any one of the preceding claims,

**characterized in that**
the two IR radiation sources (7, 11) are arranged on two opposite walls (5, 6) of the measuring chamber (2) or the two IR radiation sources (7, 11) are arranged at a distance from one another on the same wall (5) of the measuring chamber (2).

8. Device according to any one of the preceding claims,

**characterized in that**
both mirrors (5, 6) are designed as concave mirrors,
wherein the distance between the two mirrors (5, 6) and the focal lengths of the two concave mirrors (5, 6) are designed such that

- the first IR radiation source (7) is imaged in focus on the first IR detector (9) and
- the second IR radiation source (11) is imaged in focus on the second IR detector (13).

9. Device according to any one of the preceding claims,

**characterized in that**
the same two opposing walls (5, 6) of the measuring chamber (2) accommodate

- the two IR radiation sources (7, 11),
- the two IR detectors (9, 13) and
- the two mirrors (5, 6).

10. Device according to any one of the preceding claims,

**characterized in that**
the device is designed to fill the measuring chamber (2) with a gas sample not containing alcohol and to trigger the steps that

- each IR radiation source (7, 11) emits a respective IR beam into the measuring chamber (2) filled with the alcohol-free gas sample and
- each IR detector (9, 13) generates a respective zero measurement value on the basis of an IR beam incident in the case of the alcohol-free gas sample,

wherein the evaluation unit (10) is designed to make the decision about a gas sample in the measuring chamber (2) to be examined in respect of alcohol

- on the basis of measurement values which the two IR detectors (9, 13) generated while the measuring chamber (2) was filled with the gas sample to be examined and
- additionally on the basis of the two zero measurement values.

11. Method for detecting alcohol in a gas sample, in particular in exhaled respiratory air,

using a detection device which comprises

- a measuring chamber (2) for receiving a gas sample,
- a first IR radiation source (7),
- a second IR radiation source (11),
- a first IR detector (9) and
- a second IR detector (13)

wherein a respective mirror (5, 6) is arranged on each of two opposite walls of the measuring chamber (2), wherein the two IR detectors (9, 13) are arranged on the two opposing walls (5, 6) of the measuring chamber (2), wherein the method comprises the steps that

- a gas sample to be tested in respect of alcohol is brought into the measuring chamber (2),
- each of the two IR radiation sources (7, 11) emits a respective IR beam into the measuring chamber (2) while the gas sample to be tested is situated in the measuring chamber (2),
- an IR beam from the first IR radiation source (7) passes through the measuring chamber (2) and strikes the first IR detector (9),
- an IR beam from the second IR radiation source (11) passes through the measuring chamber (2) and strikes the second IR detector (13),
- each IR detector (9, 13) in each case generates at least one measurement value on the basis of an incident IR beam while the gas sample to be tested is situated in the measuring chamber (2), and
- an automated decision is made as to whether or not the gas sample in the measuring chamber (2) contains alcohol, and
- wherein the decision is made using at least the two measurement values from the two IR detectors (9, 13), and

wherein

- an IR beam emitted by the first IR radiation source (7) is reflected at least once, preferably multiple times, by at least one mirror (5, 6), before said IR beam strikes the first IR detector (9) and
- an IR beam emitted by the second IR radiation source (11) is reflected at least once, preferably multiple times, by at least one mirror (5, 6), before said IR beam strikes the second IR detector (13).

12. Method according to Claim 11,

**characterized in that**
the method comprises the further steps that

- the measuring chamber (2) is filled with an alcohol-free gas sample,
- each IR radiation source (7, 11) emits a respective IR beam into the measuring chamber (2) while the measuring chamber (2) is filled with the alcohol-free gas sample, and
- each IR detector (9, 13) respectively generates at least one zero measurement value in each case on the basis of an incident IR beam while the measuring chamber (2) is filled with the alcohol-free gas sample, and

the step of automatically deciding whether or not the gas sample to be examined in the measuring chamber (2) contains alcohol is additionally carried out using the two zero measurement values.

13. Method according to Claim 12,

**characterized in that**
the method is carried out successively for at least two different gas samples to be examined in relation to alcohol, wherein before or after the steps of

- bringing the respective gas sample to be examined into the measuring chamber (2) and
- emitting the IR beams and generating the measurement values while the gas sample to be examined is in the measuring chamber (2),

the steps that

- the alcohol-free gas sample is brought into the measuring chamber (2) and
- the IR beams are emitted, and the zero measurement values are generated while the alcohol-free gas sample is in the measurement chamber (2)

are carried out, wherein the decision as to whether or not the gas sample contains alcohol is made for each gas sample using

- measurement values that were generated while this gas sample is in the measuring chamber (2) and
- two zero measurement values from the two IR detectors (9, 13).

14. Method according to any one of Claims 11 to 13,

**characterized in that**
the step of deciding whether or not the gas sample in the measuring chamber (2) contains alcohol
is carried out using measurement values of the two IR detectors (9, 13) and additionally using a respective calibration factor for the two IR detectors (9, 13),
wherein the two calibration factors are generated automatically before the gas sample to be tested in relation to alcohol is brought into the measuring chamber (2) and
wherein the generation of the two calibration factors comprises the steps that

- the measuring chamber (2) is filled with a gas sample containing ethanol and
- the two calibration factors are generated in such a way that the two IR detectors (9, 13) have the same sensitivity to ethanol in the case of this alcohol-containing gas sample.


**Revendications**

1. Dispositif dévolu à la détection d'alcool dans un échantillon gazeux, en particulier dans de l'air pulmonaire expiré,

lequel dispositif inclut

- une chambre de mesure (2) conçue pour recevoir un échantillon gazeux,
- une première source (7) de rayonnement infrarouge,
- une seconde source (11) de rayonnement infrarouge,
- un premier détecteur d'infrarouges (9),
- un second détecteur d'infrarouges (13) et
- une unité d'évaluation (10) traitant des signaux,

un miroir (5, 6) respectif étant implanté sur deux parois de la chambre de mesure (2) mutuellement opposées,
les deux détecteurs d'infrarouges (9, 13) étant implantés sur les deux parois (5, 6) de ladite chambre de mesure (2) mutuellement opposées,
chacune des deux sources (7, 11) de rayonnements infrarouges étant agencée pour émettre, à chaque fois, un rayonnement infrarouge à destination de la chambre de mesure (2),
chacun des deux détecteurs d'infrarouges (9, 13) étant agencé pour engendrer au moins une valeur de mesure, en fonction d'un rayonnement infrarouge incident, sachant que le dispositif est agencé de façon telle

- qu'un rayonnement infrarouge, émanant de la première source (7) de rayonnement infrarouge, parcoure la chambre de mesure (2) et vienne incider sur le premier détecteur d'infrarouges (9), et
- qu'un rayonnement infrarouge, émanant de la seconde source (11) de rayonnement infrarouge, parcoure ladite chambre de mesure (2) et vienne incider sur le second détecteur d'infrarouges (13),

ledit dispositif étant agencé de telle sorte

- qu'un rayonnement infrarouge, émis par ladite première source (7) de rayonnement infrarouge, soit réfléchi au moins une fois et de préférence plusieurs fois, par au moins un miroir (5, 6), avant qu'il ne vienne incider sur ledit premier détecteur d'infrarouges (9), et
- qu'un rayonnement infrarouge, émis par ladite seconde source (11) de rayonnement infrarouge, soit réfléchi au moins une fois et de préférence plusieurs fois, par au moins un miroir (5, 6), avant qu'il ne vienne incider sur ledit second détecteur d'infrarouges (13), et

sachant que l'unité d'évaluation (10) est agencée en vue

- de décider automatiquement si un échantillon gazeux, renfermé par la chambre de mesure (2), contient de l'alcool ou n'en contient pas, et
- de prononcer cette décision en utilisant des valeurs de mesure que les deux détecteurs d'infrarouges (9, 13) ont engendrées alors même qu'un échantillon gazeux, voué à l'examen, se trouve dans ladite chambre de mesure (2).

**2.** Dispositif selon la revendication 1,
**caractérisé par le fait que**
les deux détecteurs d'infrarouges (9, 13) sont conçus pour fonctionner indépendamment l'un de l'autre.

**3.** Dispositif selon l'une des revendications précédentes,

**caractérisé par le fait que**
le premier détecteur d'infrarouges (9) comprend un premier filtre (8) de longueurs d'ondes et un premier photocapteur (24), et
le second détecteur d'infrarouges (13) comprend un second filtre (12) de longueurs d'ondes et un second photocapteur (25),
sachant que le premier filtre (8) de longueurs d'ondes est agencé pour filtrer un rayonnement infrarouge incident, de façon telle qu'un rayonnement infrarouge partiel franchisse ledit premier filtre (8) de longueurs d'ondes dans une première plage de longueurs d'ondes, et vienne incider sur le premier photocapteur (24),
sachant que le second filtre (12) de longueurs d'ondes est agencé pour filtrer un rayonnement infrarouge incident, de façon telle qu'un rayonnement infrarouge partiel franchisse ledit second filtre (12) de longueurs d'ondes dans une seconde plage de longueurs d'ondes, et vienne incider sur le second photocapteur (25), et
sachant que les deux plages de longueurs d'ondes diffèrent l'une de l'autre.

**4.** Dispositif selon la revendication 3,

**caractérisé par le fait**
**qu'**au moins l'un des deux filtres (8, 12) de longueurs d'ondes peut être actionné, sélectivement, dans un premier mode ou dans un second mode,
lequel filtre de longueurs d'ondes est agencé

- pour filtrer un rayonnement infrarouge incident, dans le premier mode, de telle manière qu'un rayonnement infrarouge partiel franchisse ledit filtre (8, 12) de longueurs d'ondes dans la première plage de longueurs d'ondes, et
- pour filtrer un rayonnement infrarouge incident, dans le second mode, de telle manière qu'un rayonnement infrarouge partiel franchisse ledit filtre (8, 12) de longueurs d'ondes dans la seconde plage de longueurs d'ondes.

**5.** Dispositif selon l'une des revendications précédentes,

**caractérisé par le fait que**
l'unité d'évaluation (10) est agencée en vue

- d'engendrer un premier signal en fonction d'une valeur de mesure du premier détecteur d'infrarouges (9) et
- d'engendrer un second signal en fonction d'une valeur de mesure du second détecteur d'infrarouges (13),

sachant que les deux signaux constituent, à chaque fois, un critère estimatif de la teneur en éthanol renfermée par un échantillon gazeux situé dans la chambre de mesure (2), et

sachant que ladite unité d'évaluation (10) est agencée, par ailleurs, en vue de comparer mutuellement lesdits deux signaux au stade de la décision visant à établir si ledit échantillon gazeux, renfermé par ladite chambre de mesure (2), contient de l'alcool ou n'en contient pas.

6. Dispositif selon la revendication 5,

   **caractérisé par le fait que**
   le dispositif de détection est agencé de façon telle que les deux signaux présentent

   - la même sensibilité à l'éthanol,
   - mais des sensibilités différentes à au moins une autre substance susceptible d'apparaître dans un échantillon gazeux renfermé par la chambre de mesure (2).

7. Dispositif selon l'une des revendications précédentes,
   **caractérisé par le fait que**
   les deux sources (7, 11) de rayonnements infrarouges sont implantées sur deux parois (5, 6) de la chambre de mesure (2) mutuellement opposées, ou bien lesdites deux sources (7, 11) de rayonnements infrarouges sont implantées, à distance l'une de l'autre, sur la même paroi (5) de ladite chambre de mesure (2).

8. Dispositif selon l'une des revendications précédentes,

   **caractérisé par le fait que**
   les deux miroirs (5, 6) sont réalisés sous la forme de miroirs concaves,
   la distance comprise entre les deux miroirs (5, 6), et les focales desdits deux miroirs concaves (5, 6), étant conçues de telle sorte que

   - la première source (7) de rayonnement infrarouge soit reproduite avec précision sur le premier détecteur d'infrarouges (9), et que
   - la seconde source (11) de rayonnement infrarouge soit reproduite avec précision sur le second détecteur d'infrarouges (13).

9. Dispositif selon l'une des revendications précédentes,
   **caractérisé par le fait que**
   les mêmes deux parois (5, 6) de la chambre de mesure (2), mutuellement opposées, reçoivent

   - les deux sources (7, 11) de rayonnements infrarouges,
   - les deux détecteurs d'infrarouges (9, 13) et
   - les deux miroirs (5, 6).

10. Dispositif selon l'une des revendications précédentes,

    **caractérisé par le fait que**
    ledit dispositif est agencé en vue d'emplir la chambre de mesure (2) à l'aide d'un échantillon gazeux exempt d'alcool, et de déclencher les étapes consistant en ce que

    - chaque source (7, 11) de rayonnement infrarouge émet, à chaque fois, un rayonnement infrarouge à destination de la chambre de mesure (2) emplie dudit échantillon gazeux exempt d'alcool, et en ce que
    - chaque détecteur d'infrarouges (9, 13) engendre, à chaque fois, une valeur de mesure nulle tributaire d'un rayonnement infrarouge venant incider au niveau dudit échantillon gazeux exempt d'alcool,

    l'unité d'évaluation (10) étant agencée en vue de prendre la décision, concernant un échantillon gazeux renfermé par ladite chambre de mesure (2) et dont l'alcool doit être contrôlé,

    - en fonction de valeurs de mesure que les deux détecteurs d'infrarouges (9, 13) ont engendrées alors même que ladite chambre de mesure (2) renferme ledit échantillon gazeux voué à l'examen, et
    - additionnellement en fonction des deux valeurs de mesure nulles.

11. Procédé de détection d'alcool dans un échantillon gazeux, en particulier dans de l'air pulmonaire expiré,

en utilisant un dispositif de détection qui inclut

- une chambre de mesure (2) conçue pour recevoir un échantillon gazeux,
- une première source (7) de rayonnement infrarouge,
- une seconde source (11) de rayonnement infrarouge,
- un premier détecteur d'infrarouges (9) et
- un second détecteur d'infrarouges (13),

un miroir (5, 6) respectif étant implanté sur deux parois de la chambre de mesure (2) mutuellement opposées, les deux détecteurs d'infrarouges (9, 13) étant implantés sur les deux parois (5, 6) de ladite chambre de mesure (2) mutuellement opposées, ledit procédé comprenant les étapes consistant

- en ce qu'un échantillon gazeux, dont l'alcool doit être contrôlé, est introduit dans la chambre de mesure (2),
- en ce que chacune des deux sources (7, 11) de rayonnements infrarouges émet, à chaque fois, un rayonnement infrarouge à destination de la chambre de mesure (2) alors même que l'échantillon gazeux, voué à l'examen, se trouve dans ladite chambre de mesure (2),
- en ce qu'un rayonnement infrarouge, émanant de la première source (7) de rayonnement infrarouge, parcourt ladite chambre de mesure (2) et vient incider sur le premier détecteur d'infrarouges (9),
- en ce qu'un rayonnement infrarouge, émanant de la seconde source (11) de rayonnement infrarouge, parcourt ladite chambre de mesure (2) et vient incider sur le second détecteur d'infrarouges (13),
- en ce que chaque détecteur d'infrarouges (9, 13) engendre, à chaque fois, au moins une valeur de mesure en fonction d'un rayonnement infrarouge incident alors même que ledit échantillon gazeux, voué à l'examen, se trouve dans ladite chambre de mesure (2), et
- en ce qu'il est décidé automatiquement si ledit échantillon gazeux, renfermé par ladite chambre de mesure (2), contient de l'alcool ou n'en contient pas,
- ladite décision étant prononcée en utilisant au moins les deux valeurs de mesure émanant des deux détecteurs d'infrarouges (9, 13),
sachant
- qu'un rayonnement infrarouge, émis par ladite première source (7) de rayonnement infrarouge, est réfléchi au moins une fois et de préférence plusieurs fois, par au moins un miroir (5, 6), avant qu'il ne vienne incider sur ledit premier détecteur d'infrarouges (9), et
- qu'un rayonnement infrarouge, émis par ladite seconde source (11) de rayonnement infrarouge, est réfléchi au moins une fois et de préférence plusieurs fois, par au moins un miroir (5, 6), avant qu'il ne vienne incider sur ledit second détecteur d'infrarouges (13).

**12.** Procédé selon la revendication 11,

**caractérisé par le fait que**
ledit procédé comprend les étapes supplémentaires consistant

- en ce que la chambre de mesure (2) est emplie d'un échantillon gazeux exempt d'alcool,
- en ce que chaque source (7, 11) de rayonnement infrarouge émet, à chaque fois, un rayonnement infrarouge à destination de la chambre de mesure (2) alors même que ladite chambre de mesure (2) est emplie de l'échantillon gazeux exempt d'alcool, et
- en ce que chaque détecteur d'infrarouges (9, 13) engendre, à chaque fois, au moins une valeur de mesure nulle respectivement tributaire d'un rayonnement infrarouge incident, alors même que ladite chambre de mesure (2) est emplie dudit échantillon gazeux exempt d'alcool, et

l'étape, consistant à décider automatiquement si l'échantillon gazeux voué à l'examen, renfermé par ladite chambre de mesure (2), contient de l'alcool ou n'en contient pas, est additionnellement exécutée en utilisant les deux valeurs de mesure nulles.

**13.** Procédé selon la revendication 12,

**caractérisé par le fait que**
ledit procédé est successivement mis en oeuvre pour au moins deux échantillons gazeux différents, dont l'alcool doit être contrôlé,

sachant que, antérieurement ou postérieurement aux étapes consistant

- à introduire, dans la chambre de mesure (2), l'échantillon gazeux respectif voué à l'examen et
- à émettre les rayonnements infrarouges et à engendrer les valeurs de mesure alors même que ledit échantillon gazeux, voué à l'examen, se trouve dans ladite chambre de mesure (2),

l'on exécute les étapes consistant

- à introduire l'échantillon gazeux exempt d'alcool dans la chambre de mesure (2) et
- à émettre les rayonnements infrarouges et à engendrer les valeurs de mesure nulles alors même que ledit échantillon gazeux, exempt d'alcool, est renfermé par ladite chambre de mesure (2),

sachant que, pour chaque échantillon gazeux, la décision visant à établir si ledit échantillon gazeux contient de l'alcool, ou n'en contient pas, est prononcée en utilisant

- des valeurs de mesure qui ont été engendrées alors même que cet échantillon gazeux se trouve dans ladite chambre de mesure (2), et
- deux valeurs de mesure nulles émanant des deux détecteurs d'infrarouges (9, 13).

14. Procédé selon l'une des revendications 11 à 13,

**caractérisé par le fait que**
l'étape de décision visant à établir si l'échantillon gazeux, situé dans la chambre de mesure (2), contient de l'alcool ou n'en contient pas,
est exécutée en utilisant des valeurs de mesure des deux détecteurs d'infrarouges (9, 13) et en utilisant additionnellement, à chaque fois, un facteur de calibrage affecté aux deux détecteurs d'infrarouges (9, 13),
sachant que les deux facteurs de calibrage sont engendrés automatiquement avant que l'échantillon gazeux, dont l'alcool doit être contrôlé, ne soit introduit dans ladite chambre de mesure (2), et
sachant que la génération desdits deux facteurs de calibrage comprend les étapes consistant

- à emplir la chambre de mesure (2) à l'aide d'un échantillon gazeux renfermant de l'éthanol, et
- à engendrer les deux facteurs de calibrage de façon telle que les deux détecteurs d'infrarouges (9, 13) aient la même sensibilité à l'éthanol en présence de cet échantillon gazeux renfermant de l'alcool.

FIG. 1

EP 3 671 184 B1

**FIG. 2**

EP 3 671 184 B1

**FIG. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011106410 B3

- US 20180116555 A1